# EUROPEAN PATENT SPECIFICATION

(11) **EP 4 331 662 B1**
(45) Date of publication and mention of the grant of the patent: **03.12.2025**
(21) Application number: 23194265.7
(22) Date of filing: 30.08.2023
(51) Int. Cl.: A61N 1/39, A61H 31/00

(54) **ADMINISTERING THERAPY BASED ON COMMUNICATION BETWEEN MEDICAL DEVICES**
VERABREICHUNG EINER THERAPIE AUF BASIS DER KOMMUNIKATION ZWISCHEN MEDIZINISCHEN VORRICHTUNGEN
ADMINISTRATION D'UNE THÉRAPIE BASÉE SUR UNE COMMUNICATION ENTRE DES DISPOSITIFS MÉDICAUX

(30) Priority: 31.08.2022 US 202263402776 P
(43) Date of publication of application: 06.03.2024
(73) Proprietor: Stryker Corporation, Portage, MI 49002 (US)
(72) Inventor: GREENEWALD, Robert, Kalamazoo, 49002 (US); APPERSON, Ryan W., Kalamazoo, 49002 (US); CHAPMAN, Fred W., Kalamazoo, 49002 (US); LAGERSTROM, Jonas, Kalamazoo, 49002 (US); LINDROTH, Sara, Kalamazoo, 49002 (US); RUTZER, Mark, Kalamazoo, 49002 (US); SANDRUP, Eric, Kalamazoo, 49002 (US); SVAHN, Tobias, Kalamazoo, 49002 (US)
(74) Representative: V.O.

(56) References cited:
- EP-A2- 1 913 922
- DE-U1- 202008 014 773
- US-A1- 2008 114 406
- US-A1- 2018 256 446

## Description

### BACKGROUND

Medical devices can be used to facilitate patient monitoring, facilitate patient treatments, or a combination of both. In some environments, multiple medical devices are located in close proximity to one another. Some may be monitoring and/or treating the same patient, and some may be monitoring and/or treating different patients. For example, EP1913922, US2008114406, US2018256446 and DE202008014773 describe cooperating chest compression devices and defibrillators.

### SUMMARY

The invention is defined in claim 1. Further advantageous embodiments are defined in dependent claims 2-5 Various implementations described herein relate to techniques for assisting in administering therapy and for monitoring a subject (e.g., a patient) based on communication between medical devices. In some examples, multiple medical devices are managing care of the same patient. For instance, a monitoring device detects a condition of the patient and a treating device administers a treatment to the patient. In some examples, the monitoring device is also configured to administer treatment to the patient. The treatment, from either or both devices, is enhanced when the devices coordinate with one another. In particular examples, the devices coordinate with each other by exchanging messages over a wireless interface. The messages, for instance, include reports about the condition of the patient, reports about ongoing treatments to the patient, as well as instructions to perform actions (e.g., additional treatments).

However, when multiple medical devices are associated with a patient, conflicts may arise between the multiple medical devices. For example, it may be difficult for the monitoring device to adequately monitor a condition(s) of the patient while the treating device is administering treatment to the patient. As another example, it may be difficult, impracticable, and/or inadvisable for multiple medical devices to administer treatment to the patient at the same time. In high-stress, chaotic environments, it may be challenging for a single user to operate multiple medical devices in an effective and safe manner. Moreover, if the user stops the operation of a first medical device in order to operate a second medical device (e.g., to monitor and/or treat a subject), inefficiencies may arise due to the downtime associated with the first medical device. In other words, while the first medical device is not operating to monitor and/or treat the subject, the subject does not benefit from the first medical device, which could be monitoring and/or treating the subject were it not for the difficulties and safety risks involved in manually operating multiple medical devices at once. Accordingly, the techniques, devices, and systems described herein allow multiple medical devices that are monitoring and/or treating the same subject to operate in coordination with one another in order to minimize conflicts between the medical devices, minimize the downtime of a given medical device, and/or increase the efficiency of monitoring the subject and/or administering therapy(ies) to the subject using the multiple medical devices.

According to various implementations of the present disclosure, data may be transmitted between paired medical devices. This data may be analyzed (e.g., by the recipient medical device) to determine a parameter(s) for controlling the therapy that is to be administered by one or more of the medical devices. In this manner, therapy(ies) can be administered by one or more of the medical devices in accordance with the control parameter(s). For example, a monitor-defibrillator may receive data from a mechanical chest compression device that is administering chest compressions to a subject. The monitor-defibrillator can analyze this data to determine a control parameter(s) for controlling administration of a therapy (e.g., pacing, defibrillation, etc.) to the subject by the monitor-defibrillator. The monitor-defibrillator can analyze this data for controlling administration of a therapy (e.g., pacing, defibrillation, etc.) to the subject by the monitor-defibrillator. The monitor-defibrillator can e.g. generate a control parameter(s) to control delivery of an electrical shock to the subject in coordination with the chest compressions that are being administered to the subject, such as to request delivering the electrical shock during an upcoming pause or interval between sequential chest compressions. The monitor-defibrillator can generate a control signal configure to cause administering of the therapy to the subject in accordance with the control parameter(s).The monitor-defibrillator can administer the therapy to the subject in accordance with the control parameter(s). The monitor-defibrillator can, for instance, deliver an electrical shock to the subject in coordination with the chest compressions that are being administered to the subject, such as by delivering the electrical shock during an upcoming pause or interval between sequential chest compressions. In this example, the downtime of the chest compression device can be reduced, if not eliminated through the coordination between the medical devices, and the subject is provided with care in an efficient manner using the multiple medical devices because the mechanical chest compression device does not have to be interrupted (e.g., by ceasing to administer chest compressions) in order to utilize the monitor-defibrillator to monitor and/or treat the subject.

As another example, a mechanical chest compression device may receive data from a monitor-defibrillator that is administering therapy to a subject. The chest compression device can analyze the data to determine a control parameter(s) for controlling administration of chest compressions to the subject by the chest compression device. The chest compression device can, for instance, generate a control parameter(s) to control administering chest compressions at a frequency, timing, duty cycle, depth, force, velocity, acceleration, height, and/or position with respect to the subject based on data (e.g., physiological parameter data) received from the monitor-defibrillator. The chest compression device can generate a control signal configured to cause administering of the chest compressions to the subject in accordance with the control parameter(s). The chest compression device can administer the chest compressions to the subject in accordance with the control parameter(s). In this manner, the mechanical chest compression device can, for instance, administer chest compressions at a frequency, timing, duty cycle, depth, force, velocity, acceleration, height, and/or position with respect to the subject based on data (e.g., physiological parameter data) received from the monitor-defibrillator. In this manner, the mechanical chest compression device can adjust the administration of chest compressions on-the-fly, without waiting for an operator (e.g., a rescuer) to make the adjustments manually.

According to an aspect, not part of the claimed invention but useful for the general understanding of the invention, it is provided a method comprising receiving, by an external defibrillator, data from a mechanical chest compression device that is administering chest compressions to a subject; determining, by the external defibrillator analyzing the data, a timing for administering a therapy to the subject in coordination with the chest compressions; and generating, by the external defibrillator, a control signal configured to cause the external defibrillator to administer the therapy to the subject in accordance with the timing.

Optionally, the therapy comprises defibrillation. Alternatively, or additionally, the therapy comprises pacing.

Optionally, the method comprises determining, by the external defibrillator analyzing the data, a time of an upcoming pause that will follow one of the chest compressions administered to the subject by the mechanical chest compression device; and detecting, by the external defibrillator, a physiological parameter of the subject during the time.

Optionally, the method comprises determining, by the external defibrillator analyzing the data, a medication to administer the subject; and outputting, by the external defibrillator, an indication of the medication.

Optionally, the method comprises determining, by the external defibrillator analyzing the data, a frequency of the chest compressions; identifying, by the external defibrillator, a filter characterized by the frequency; and removing a chest compression artifact from physiological data representing a physiological parameter of the subject by applying the filter to the physiological data.

Optionally, the method comprises determining, by the external defibrillator analyzing the data, a position with respect to the subject at which the chest compressions are being administered; and outputting, by the external defibrillator, an indication of the position.

Optionally, the method comprises determining, by the external defibrillator analyzing the data, an alarm associated with the mechanical chest compression device; and outputting, by the external defibrillator, an indication of the alarm.

Optionally, the method comprises determining, by the external defibrillator analyzing the data, a level at which an electrical shock is to be delivered to the subject by the external defibrillator, wherein the control signal is configured to cause delivering, by the external defibrillator, the electrical shock at the level to the subject.

According to the invention it is provided an external defibrillator comprising: a transceiver;
a discharge circuit configured to output an electrical shock to a subject; and a processor configured to: receive, via the transceiver, data from a mechanical chest compression device;
determine, by analyzing the data, a timing for outputting an electrical shock to the subject in coordination with chest compressions that are being administered to the subject by the mechanical chest compression device. The processor can be configured to generate a control signal configured to cause the discharge circuit to output the electrical shock to the subject in accordance with the timing. The processor can be configured to cause the discharge circuit to output the electrical shock to the subject in accordance with the timing.

Optionally, the electrical shock comprises a defibrillation shock.

The invention is further characterized in that the external defibrillator comprises an output device. The output device is configured to output information, wherein the processor is further configured to: determine, by analyzing the data, a time of an upcoming pause that will follow a chest compression administered to the subject by the mechanical chest compression device; detect a physiological parameter of the subject only during the time; and cause the output device to output the physiological parameter. Optionally, the output device is configured to output information, wherein the processor is further configured to: determine, by analyzing the data, a medication for the subject; and cause the output device to output an indication of the medication. Optionally, the output device is configured to output information, wherein the processor is further configured to: determine, by analyzing the data, a depth to which the chest compressions are being administered; and cause the output device to output an indication of the depth. Optionally, the output device is configured to output information, wherein the processor is further configured to: determine, by analyzing the data, a force with which the chest compressions are being administered; and cause the output device to output an indication of the force.

### BRIEF DESCRIPTION OF THE DRAWINGS

FIGS. 1A and 1B illustrate example environments for administering therapy to a subject at a rescue scene based on communication between medical devices.
FIG. 2 illustrates an example environment in which a monitoring device and a treating device are configured to communicate via one or more connections.
FIG. 3 illustrates example information output via an output device of a medical device based on communication with another medical device.
FIG. 4 illustrates an example process for administering therapy by a first medical device based on data received from a second medical device.
FIG. 5 illustrates an example process for coordinating the detection of a physiological parameter of a subject by a first medical device during a pause in therapy that a second medical device is administering to the subject.
FIG. 6 illustrates an example process for outputting information via an output device of a first medical device by analyzing data received from a second medical device.
FIG. 7 illustrates an example process for pausing therapy that a second medical device is administering to a subject in order to detect a parameter associated with therapy that is being administered to the subject by a first medical device, and analyzing the parameter to determine how to coordinate therapies administered by the medical devices.
FIG. 8 illustrates an example of an external defibrillator configured to perform various functions described herein.
FIG. 9 illustrates a chest compression device configured to perform various functions described herein.

### DETAILED DESCRIPTION

It is to be appreciated that, while several of the example described herein pertain to a monitor-defibrillator (e.g., external defibrillator) and a mechanical chest compression device that communicate with one another to provide coordinated care to a subject (e.g., a patient), the techniques described herein may be implemented with respect to other types of paired medical devices. Moreover, other techniques for monitoring and/or administering therapy to a subject (e.g., a patient) based on communication between medical devices are also within the scope of the present disclosure.

Implementations of the present disclosure are directed to improvements in the technical field of medical devices. Rather than manually operating multiple medical devices independently of one another to treat a subject (e.g., a patient), implementations described herein enable automated communication and coordination between paired medical devices to minimize conflicts between the medical devices, minimize downtime of the medical devices, and increase the efficiency of monitoring and therapy performed by the multiple medical devices.

Various examples will now be described with reference to the accompanying drawings.

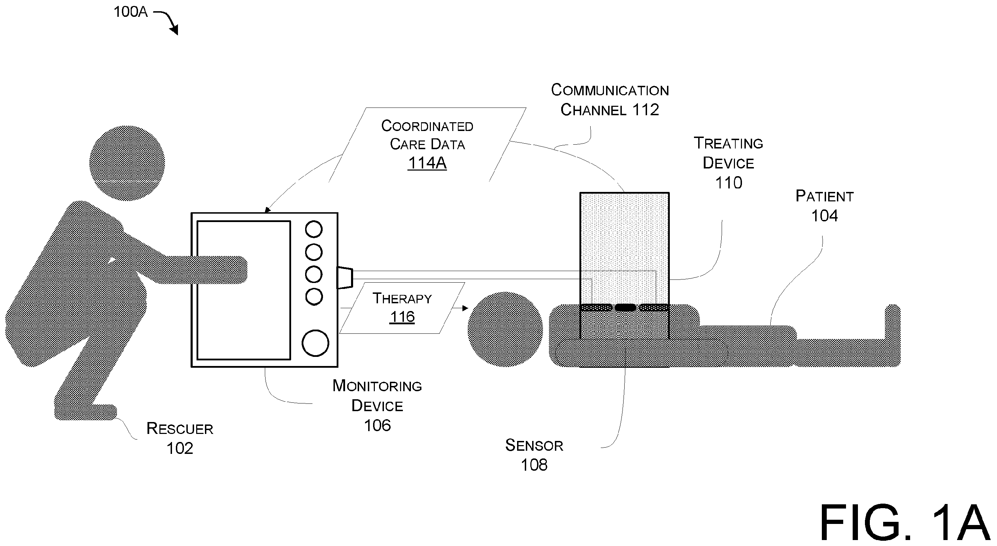

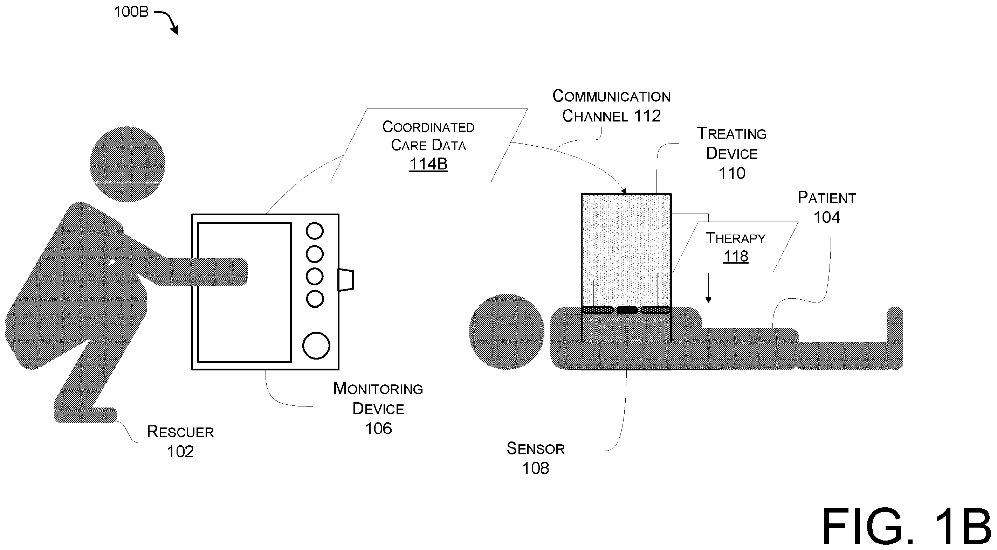

FIGS. 1A and 1B illustrate example environments for administering therapy to a subject at a rescue scene based on communication between medical devices. The rescue scene, in some implementations, is within a clinical environment, such as a hospital or other managed care facility. In some cases, the rescue scene is outside of a clinical environment.

FIG. 1A illustrates a rescue scene 100A. In various implementations, a rescuer 102 is treating a patient 104 at the rescue scene 100A. For instance, the rescuer 102 is an EMT professional monitoring and/or treating a medical condition of the patient 104. In some cases, the patient 104 is experiencing cardiac arrest or some other dangerous medical condition.

The rescuer 102 is monitoring the condition of the patient 104 using a monitoring device 106. For instance, the monitoring device 106 may be a monitor-defibrillator, a medical imaging device, an ultrasound monitor, a standalone electrocardiogram (ECG) monitor, or another type of patient monitor. The monitoring device 106 includes and/or is communicatively coupled to a sensor 108. The sensor 108 is configured to detect at least one physiological parameter of the patient 104. Examples of physiological parameters include, for instance, an ECG, an impedance, a force administered to the patient 104, a blood pressure, an airway parameter (e.g., a partial pressure of carbon dioxide, a partial pressure of oxygen, a capnograph, an end tidal gas parameter, a flow rate, etc.), a blood oxygenation (e.g., a pulse oximetry value, a regional oximetry value, etc.), an electroencephalogram (EEG), a temperature, a heart sound, a blood flow rate, a physiological geometry (e.g., a shape of a blood vessel, an inner ear shape, etc.), a heart rate, a pulse rate, or the like. For example, the sensor 108 includes at least one of electrodes, a detection circuit, defibrillator pads, a force sensor, a blood pressure cuff, an ultrasound-based blood pressure sensor, an invasive (e.g., intra-arterial) blood pressure sensor (e.g., including a cannula inserted into the patient 104), a gas sensor (e.g., a carbon dioxide and/or oxygen sensor), a flowmeter, a pulse oximetry sensor a regional oximetry sensor, a thermometer, a microphone, an ultrasound transducer, a medical imaging device (e.g., an ultrasound imaging device), or the like. In various cases, the monitoring device 106 outputs the physiological parameter(s) to the rescuer 102. For instance, the monitoring device 106 includes a display, a speaker, or haptic feedback device that conveys the physiological parameter(s) to the rescuer 102.

A treating device 110 (also referred to as a "treatment device") is configured to administer a treatment (e.g., therapy) to the patient 104. For example, the treating device 110 is a monitor-defibrillator, an automated external defibrillator (AED), mechanical chest compression device, a smart bag-valve mask, a ventilator, a heart-lung machine, an intravenous fluid (IV) pump, or the like. Examples of treatments (e.g., therapies) include defibrillation, pacing, cardioversion, administration of chest compressions, administration of oxygen to the airway of the patient 104, movement of air in the airway of the patient, administration of fluids to the patient 104, extracorporeal membrane oxygenation (ECMO), administration of a medication to the patient 104, or the like. In some implementations, the monitoring device 106 is also configured to administer a treatment to the patient 104. Further, in some cases, the treating device 110 is configured to detect one or more physiological parameters of the patient 104.

In some cases, the monitoring and/or treatment of the patient 104 is optimized by communication between the monitoring device 106 and the treating device 110. In particular examples, the monitoring device 106 or the treating device 110 reports a detected physiological parameter to the other device. For instance, the treating device 110 may include a blood pressure sensor and may report a blood pressure of the patient 104 to the monitoring device 106. In some cases, the monitoring device 106 or the treating device 110 reports a treatment parameter to the other device. For example, the treating device 110 may report, to the monitoring device 106, a frequency of chest compressions administered by the treating device 110. The receiving device may perform one or more actions based on the physiological parameter and/or the treatment parameter. Actions performed by the monitoring device 106 or the treating device 110 can include initiating a measurement of a physiological parameter at a particular time or frequency, outputting a signal to the rescuer 102, outputting a signal to the patient 104, generating an instruction for performing a treatment at a particular time or frequency, generating an instruction for adjusting a treatment parameter of an ongoing treatment, or any combination thereof. According to some implementations, the monitoring device or the treating device 110 transmits an instruction the other device to perform one or more actions. The receiving device is configured to perform the action(s) based on the instruction from the monitoring device 106 or the treating device 110. The receiving device can generate an internal control signal to perform the action(s) based on the instruction from the monitoring device 106 or the treating device 110. For instance, the monitoring device 106 instructs the treating device 110 to cease administering chest compressions to the patient 104 at a predetermined time, and the monitoring device 106 administers a defibrillation shock to the patient 104 at the particular time. By exchanging reports, instructions, or other data, the monitoring device 106 and the treating device 110 can coordinate monitoring and treatment of the patient 104.

To exchange data, the monitoring device 106 and/or the treating device 110 are configured to establish and/or communicate via a communication channel 112. As used herein, the term "communication channel," and its equivalents, may refer to a medium over which a first endpoint (e.g., a sender) transmits information to one or more second endpoints (e.g., receivers). Examples of communication channels include wired connections, such as Ethernet or fiber optic paths, as well as wireless connections, such as Institute of Electronics and Electrical Engineers (IEEE) (e.g., WI-FI, BLUETOOTH, etc.) or 3^{rd} Generation Partnership Program (3GPP) (e.g., Long Term Evolution (LTE), New Radio (NR), etc.) connections. As used herein, the term "endpoint," and its equivalents, may refer to an entity that is configured to transmit and/or receive data. Examples of endpoints include user equipment (UE) (e.g., mobile phones, tablet computers, etc.), computers, base stations, access points (APs), servers, compute nodes, medical devices, Internet of Things (IoT) devices, and the like.

In some implementations, the communication channel 112 between the monitoring device 106 and the treating device 110 is established when the monitoring device 106 and the treating device 110 are paired. In particular cases, the monitoring device 106 and treating device 110 refrain from sharing substantive data (e.g., physiological metrics, reports about the patient 104, instructions for treating the patient 104, etc.) until the monitoring device 106 and the treating device 110 are paired. As used herein, the term "paired," and its equivalents, may refer to a state of multiple devices that have a shared link key that enables each device to cryptographically authenticate data it receives from any other device among the multiple devices.

Various mechanisms can be utilized to pair the monitoring device 106 with the treating device 110. For example, automated pairing may involve the exchange of data and/or pairing requests/responses between the devices 106 and 110. As another example, the monitoring device 106 may receive an input signal from an operator (e.g., the rescuer 102) that selects the treating device 110 as a device to pair with the monitoring device 106, or vice versa. In some cases, the monitoring device 106 detects an alternative signal (e.g., a flashing light pattern) from the treating device 110 that is indicated in a pairing request. In some cases, the monitoring device 106 and the treating device 110 are paired, at least in part, based on signaling to and/or from an intermediary device (not illustrated in FIGS. 1A and 1B). In a particular example, two or more devices can be brought into proximity to (e.g., into contact with) each other in order to facilitating pairing the monitoring device 106 with the treating device 110. For example, a "tap-to-pair" functionality may allow a user to bring the monitoring device 106 (or a component thereof) into close proximity to (e.g., into contact with) the treating device 110 (or a component thereof), or vice versa, and a short-range wireless protocol, such as BLUETOOTH, near-field communication (NFC), or the like, may be used to detect that the devices 106 and 110 are within a threshold distance of each other, and, in response, the devices 106 and 110 may be paired. In some cases, an intermediary device, such as a phone, may be brought into close proximity to (e.g., into contact with) the monitoring device 106 and/or the treating device 110 (e.g., by touching the intermediary device to both devices 106 and 110 sequentially), and a short-range wireless protocol may be used to detect these proximity events involving the intermediary device, and, in response, the devices 106 and 110 may be paired.

In particular cases, a first paired device encrypts data prior to transmitting the data to a second paired device, and the second paired device restores the original data by decrypting the encrypted data. As used herein, the term "encrypt," and its equivalents, refers to a process of translating data from one format (e.g., an unencoded format) into an encoded format. In various cases, the encoded format is referred to as "ciphertext." Unencoded data, which has not been encrypted, may be referred to as being in "plaintext." In various examples, an entity encrypts data using at least one encryption key. An encryption key is a parameter that defines the translation of data from the one format into the encoded format. As used herein, the term "decrypt," and its equivalents, refers to a process of translating data from an encoded format into another format (e.g., an unencoded format), such as a plaintext format. In various examples, an entity encrypts data using at least one decryption key. A decryption key is a parameter that defines the translation of data from the encoded format into the other format. A link key, for example, is an encryption and/or decryption key.

Various cryptographic techniques can be utilized in accordance with the features described in this disclosure. For example, data can be encrypted and decrypted via a symmetric key, wherein the encryption key and the decryption key are equivalent. In some cases, data can be encrypted and decrypted via asymmetric keys, wherein the encryption key and the decryption key are different. Cryptographic hash functions (CHFs) are examples of cryptographic techniques. Examples of cryptographic techniques include the Data Encryption Standard (DES), Advanced Encryption Standard (AES), Elliptic Curve Cryptography (ECC), Rivest-Shamir-Adleman (RSA), Secure Hash Algorithm (SHA)-1, SHA-2, SHA-3, BLAKE, BLAKE2, BLAKE3, WHIRLPOOL, MD2, MD4, MD5, MD6, Temporal Key Integrity Protocol (TKIP), Rivest cipher 4 (RC4), variably modified permutation composition (VMPC), blowfish, Twofish, Threefish, Tiny Encryption Algorithm (TEA), Extended TEA (XTEA), Corrected Block TEA (XXTEA), Diffie-Hellman exchange (DHE), elliptic curve DHE, supersingular isogeny Diffie-Hellman (SIDH) key exchange, and so on. Any suitable encryption or decryption technique can be used in accordance with implementations of this disclosure.

In FIGS. 1A and 1B, because the monitoring device 106 and the treating device 110 are paired, the monitoring device 106 and the treating device 110 establish the communication channel 112 and exchange coordinated care data 114 over the communication channel 112. The coordinated care data 114 includes substantive data relevant to monitoring and/or treating the patient 104. In some implementations, the coordinated care data 114 indicates one or more physiological parameters of the patient 104. For example, the treating device 110 may detect a physiological parameter of the patient 104 and report the physiological parameter to the monitoring device 106. In some implementations, the monitoring device 106 outputs the physiological parameter to the rescuer 102, such as on a display of the monitoring device 106. In some cases, the monitoring device 106 determines a condition of the patient 104 based on the physiological parameter detected by the treating device 110. For instance, the treating device 110 may detect and report a blood pressure of the patient 104 to the monitoring device 106 in the coordinated care data 114, and the monitoring device 106 may determine whether the patient 104 has return of spontaneous circulation (ROSC) by analyzing the blood pressure and the ECG of the patient 104.

In various cases, the coordinated care data 114 includes a treatment parameter associated with a treatment (e.g., therapy) that is being administered by the monitoring device 106 and/or the treating device 110. For example, the treating device 110 may report, to the monitoring device 106, a frequency and/or timing of chest compressions that are being administered to the patient 104, a position with respect to the patient 104 at which the chest compressions are being administered, a depth to which the chest compressions are being administered to the patent 104, and/or a force with which the chest compressions are being administered to the patent 104, among other treatment parameters described herein.

In various cases, the coordinated care data 114 includes one or more instructions. The monitoring device 106, for instance, instructs the treating device 110 to detect a physiological parameter of the patient 104, to administer a treatment to the patient 104, to pause or cease administering a treatment to the patient 104, to modify a treatment of the patient 104, or any combination thereof. In some cases, the treating device 110 instructs the monitoring device 106 to detect a physiological parameter of the patient, to administer a treatment to the patient 104, to pause or cease administering a treatment to the patient 104, to modify a treatment of the patient 104, or any combination thereof.

According to some examples, the coordinated care data 114 includes synchronization data that enables the monitoring device 106 and the treating device 110 to synchronize clocks. As used herein, the term "clock," and its equivalents, may refer to hardware and/or software of a device that tracks time. In various examples, the monitoring device 106 includes a first clock and the treating device 110 has a second clock. Before the monitoring device 106 and the treating device 110 are paired, their clocks may be unsynchronized, such that the devices may perceive a particular time at different actual times. In some implementations, the coordinated care data 114 includes data that enables the monitoring device 106 and the treating device 110 to be able to perceive a particular time at the same actual time. For example, if the monitoring device 106 instructs the treating device 110 to administer a chest compression at a particular time in the future, the synchronized clocks enable the treating device 110 to actually administer the chest compression at the particular time.

A particular example will now be described with reference to FIG. 1A, wherein the monitoring device 106 is a monitor-defibrillator and the treating device 110 is a mechanical chest compression device. The monitoring device 106 and the treating device 110 are paired, as indicated by the established communication channel 112. The treating device 110 is administering chest compressions to the patient 104. While administering the chest compressions, the treating device 110 sends data 114A to the monitoring device 106, and the monitoring device 106 receives the data 114A from the treating device 110. The monitoring device 106 analyzes this data 114A to perform one or more actions, as described in the various examples herein. In the example of FIG. 1A, the monitoring device 106 determines a control parameter(s) by analyzing the data 114A. The control parameter(s) may be determined for controlling the administration of a therapy 116 to the patient 104 by the monitoring device 106. Accordingly, FIG. 1A depicts the monitoring device 106 administering the therapy 116 to the patient 104. In this example, the motoring device 106 generates an internal control signal for administering the therapy 116 based on the control parameter(s). In this example, the therapy 116 is administered in accordance with the control parameter(s) determined by analyzing the data 114A received from the treating device 110.

In some examples, the control parameter(s) determined by the monitoring device 106 of FIG. 1A is indicative of a timing with which the therapy 116 is to be administered to the patient 104 by the monitoring device 106. The internal control signal for administering the therapy 116 can be indicative of a timing with which the therapy 116 is to be administered to the patient 104 by the monitoring device 106. In this manner, the therapy 116 can be administered by the monitoring device 106 in accordance with the timing in order to coordinate the administration of the therapy 116 with the chest compressions that are being administered by the treating device 110. For example, the monitoring device 106 may determine, by analyzing the data 114A, a timing for administering the therapy 116 to the patient 104 that occurs during an upcoming pause or interval between sequential chest compressions. In this example, the data 114A received by the monitoring device 106 may indicate the timing and/or frequency of the chest compressions. If the respective clocks of the devices 106 and 110 are synchronized, as described herein, the therapy 116 can be administered during a time period that corresponds to a pause, or an interval, between sequential chest compressions administered by the treating device 110. In some examples, the control parameter(s) determined by the monitoring device 106 of FIG. 1A is a shock parameter indicative of a level at which an electrical shock is to be delivered to the patient 104 by the monitoring device 106.

The therapy 116 to be administered by the monitoring device 106 can be defibrillation, pacing, or another suitable type of therapy. Defibrillation therapy involves outputting, via a discharge circuit, an electrical shock to the patient 104, where the electrical shock is designed to help the patient 104 recover from a life-threatening cardiac arrhythmia. Pacing therapy involves outputting, via a discharge circuit, electrical shocks (or pacing pulses) to the patient 104, wherein the electrical shocks are designed to control and/or modify a rate at which the heart beats.

In an example where the therapy 116 represents defibrillation, the output of a defibrillation shock by the monitoring device 106 can be coordinated with a pause or interval between sequential chest compressions, in which case the pause/interval duration and/or the timing of the chest compressions can be optimized in relation to shock delivery. For example, the treating device 110 may generate a control signal to adjust the timing and/or frequency of chest compressions being administered in preparation for the delivery of a defibrillation shock to the patient 104 by the monitoring device 106. In some examples, one or more defibrillation shocks can be output by the monitoring device 106 during ongoing and continued chest compressions administered by the treating device 110, such as by delivering a defibrillation shock to the patient 104 relative to a chest compression duty cycle of the treating device 110, and/or relative to an upstroke duty cycle and/or a downstroke duty cycle of the treating device 110.

In an example where the therapy 116 represents pacing, a frequency at which pacing pulses are output by the monitoring device 106 can be coordinated with chest compressions being administered by the treating device 110. This coordinated treatment may be useful if the sensor 108, for instance, detects that the patient 104 is exhibiting pulseless electrical activity (PEA), bradycardia (e.g., slow cardiac rhythm and/or weak contractions), asystole with p-waves, and/or low blood pressure (e.g., below a threshold blood pressure).

In the preceding examples with respect to FIG. 1A, the control signal can be configured such that the therapy 116 can be administered by the monitoring device 106 without stopping or interrupting the treatment (e.g., chest compressions) that is being administered the treating device 110. Minimizing, if not eliminating, the downtime of the treating device 110 while monitoring the patient 104 and/or administering therapy via the monitoring device 106 can benefit the patient 104 and improve the patient 104 outcome. For instance, the ability of the treating device 110 to continue chest compressions as part of CPR therapy without interruption notwithstanding the monitoring and/or administration of therapy 116 (e.g., defibrillation, pacing, etc.) by the monitoring device 106 can, in some cases, result in the patient 104 recovering from a life-threatening medical event, such as a life-threatening cardiac arrhythmia (e.g., ventricular fibrillation (VF)).

With reference to FIG. 1B, another particular example will now be described, wherein the monitoring device 106 is a monitor-defibrillator and the treating device 110 is a mechanical chest compression device. The monitoring device 106 and the treating device 110 are paired, as indicated by the established communication channel 112. The monitoring device 106 is administering therapy to the patient 104, such as defibrillation, pacing, or another suitable therapy. While administering the therapy, the monitoring device 106 sends data 114B to the treating device 110, and the treating device 110 receives the data 114B from the monitoring device 106. The treating device 110 analyzes this data 114B to perform one or more actions, as described in the various examples herein. In the example of FIG. 1B, the treating device 110 determines a control parameter(s) by analyzing the data 114B. The control parameter(s) may be determined for controlling the administration of a therapy 118 to the patient 104 by the treating device 110. Accordingly, FIG. 1B depicts the treating device 110 administering the therapy 118 to the patient 104. In this example, the treating device 110 generates an internal control signal for administering the therapy 118 based on the control parameter(s). In this example, the therapy 118 is administered in accordance with the control parameter(s) determined by analyzing the data 114B received from the monitoring device 106.

In some examples, the data 114B received from the monitoring device 106 is indicative of a physiological parameter associated with the patient 104. For example, the sensor 108 may have been used to determine an end tidal gas parameter (e.g., ETCO₂) associated with the patient 104, a blood oxygenation associated with the patient 104, a blood pressure associated with the patient 104, an ECG of the patient 104, and/or an intrinsic heartbeat of the patient, and the data 114B may be indicative of any one, or combination of, such physiological parameters. By analyzing such data 114B, the treating device 110 may determine a control parameter(s) associated with the therapy 118 (e.g., chest compressions) that is to be administered to the patient 104, such as a frequency, timing, duty cycle, depth, force, velocity, acceleration, height, and/or position of the chest compressions that are to be administered to the patent 104. In this manner, the therapy 118 can be administered by the treating device 110 in accordance with the control parameter(s), such as to improve the outcome of the patent 104.

In an illustrative example, the treating device 110 may determine, by analyzing the data 114B, a frequency at which to administer chest compressions to the patient 104. The internal control signal for administering chest compressions can be indicative of the frequency. The chest compressions can be administered to the patient 104 at the frequency by the treating device 110, as depicted in FIG. 1B by the administration of the therapy 118. For example, if the data 114B is indicative of the intrinsic heartbeat and/or the ECG of the patient 104, the treating device 110 may coordinate the frequency of the chest compressions with the intrinsic heartbeat of the patient 104 (e.g., the frequency of the chest compressions may match the frequency of the intrinsic heartbeat). This may be beneficial if the patient 104 is in circulatory shock, for example.

In another example, the treating device 110 may determine, by analyzing the data 114B, a position with respect to the patient 104 at which chest compressions are to be administered. The internal control signal can be indicative of the position. The chest compressions can be administered to the patient 104 by the treating device 110 at the position (e.g., at a particular position on the chest of the patient 104). In this example, the data 114B may indicate that the patient 104 could benefit from chest compressions administered at a different position on the chest of the patient 104, as compared to the current position at which the chest compressions are being administered. Accordingly, the treating device 110 may include a mechanism (e.g., a motor(s) and gear assembly) to automatically adjust the position of a component (e.g., a compressor) of the treating device 110 that is pressing and/or pulling on the patient's 104 chest for administering the therapy 118.

In another example, the treating device 110 may determine, by analyzing the data 114B, a depth to which the chest compressions are to be administered to the patient 104. The internal control signal can be indicative of the depth. The chest compressions can be administered to the patient 104 by the treating device 110 to the determined depth. Because the treating device 110 may be configured to actively decompress the patient's chest, such as by pulling up (or outward) on the chest to expand the chest cavity, the treating device 110 may, for instance, determine, by analyzing the data 114B, a height to which such active decompressions are to be administered to the patient 104, and the active decompressions can be administered to the patient 104. It is to be appreciated that the terminology "chest compressions," as used herein, can include active decompressions where the chest compression device pulls the chest outward.

In some examples, the data 114B received from the monitoring device 106 in the example of FIG. 1B represents a control parameter(s) for controlling the administration of the therapy 118 by the treating device 110. In other words, the monitoring device 106 may determine how the therapy 118 is to be administered by the treating device 110, and may communicate corresponding control data to the treating device 110. For example, the data 114B may represent a frequency, timing, duty cycle, depth, force, velocity, acceleration, height, and/or position of the chest compressions that are to be administered by the treating device 110 as the therapy 118 in FIG. 1B. The monitoring device 106 can determine such a control parameter(s) to communicate via the data 114B in any suitable way, such as by analyzing a parameter associated with the therapy that is being administered to the patient 104 by the monitoring device 106. Such a parameter may be a physiological parameter of the patient 104, as described herein. For example, if the sensor 108 is used by the monitoring device 106 to detect the intrinsic heartbeat and/or the ECG of the patient 104, the data 114B sent from the monitoring device 106 to the treating device 110 may represent a frequency of the chest compressions. By analyzing the data 114B, the treating device 110 may generate a control signal to coordinate the frequency of the chest compressions with the intrinsic heartbeat of the patient 104. The treating device 110 may administer the chest compressions at the frequency in order to coordinate the frequency of the chest compressions with the intrinsic heartbeat of the patient 104.

Accordingly, the monitoring device 106 and the treating device 110 are configured to coordinate their monitoring and/or treatment in the various exemplary ways described herein to minimize conflicts between the devices 106 and 110, minimize downtime of the device 106 and/or the device 110, and/or increase the efficiency of monitoring and therapy performed by the devices 106 and/or 110. For instance, in some cases, the patient 104 may be harmed if the monitoring device 106 administers an electrical shock while the treating device 110 is administering chest compressions. Accordingly, if, for instance, the monitoring device 106 determines that the patient 104 is experiencing ventricular fibrillation, VF, by analyzing the ECG of the patient 104, in order to avoid the potential for injury to the patient 104, the monitoring device 106 can coordinate the delivery of a defibrillation shock with ongoing chest compressions (e.g., by controlling administering the defibrillation therapy 116 during an upcoming pause or interval between sequential chest compressions), and the timing and/or duration of such a pause/interval can be optimized for the administration of such defibrillation therapy 116. In other examples, the monitoring device 106 may send an instruction to the treating device 110 instructing the treating device 110 to pause or cease administration of chest compressions while the monitoring device 106 administers the electrical shock.

In another particular example, the monitoring device 106 is a patient monitor and the treating device 110 is an extra corporeal membrane oxygenation (ECMO) or other type of extra corporeal life support (ECLS) device that removes carbon dioxide from the blood of the patient 104, increases oxygen in the blood of the patient 104, and pumps the blood through the body of the patient 104. In some instances, the monitoring device 106 determines that a peripheral oxygenation of the patient 104 is below a threshold. In response, the monitoring device 106 transmits an instruction in the data 114B to increase the oxygenation rate of the treating device 110 (or to otherwise adjust the sweep of the device). The treating device 110 can generate a control signal for administering the therapy 118 in accordance with the instruction from the monitoring device 106. The treating device 110 can administer the therapy 118 in accordance with the instruction from the monitoring device 106.

In a further example, the monitoring device 106 is an ultrasound monitor and the treating device 110 is a ventilation device configured to introduce oxygen to the airway of the patient 104 at a particular respiration rate. An ultrasound transducer of the monitoring device 106 is configured to emit ultrasound into the patient 104 (e.g., at a neck of the patient 104) and to detect ultrasound scattered from the patient 104. The monitoring device 106 generates an image of a portion of the patient 104 by analyzing the emitted and detected ultrasound. In some cases, the monitoring device 106 detects that the patient 104 is experiencing gastric insufflation. In response, the monitoring device 106 transmits an instruction in the data 114B to the treating device 110.the treating device 110 can generate a control signal to lower a flow rate of air (including oxygen) to be administered to the patient 104 in accordance with the instruction from the monitoring device. The treating device 110 can lowers a flow rate of air (including oxygen) administered to the patient 104 in accordance with the instruction from the monitoring device 106.

In FIGS. 1A and 1B, the monitoring device 106 and treating device 110 are illustrated as communicating directly. However, implementations are not so limited. For example any illustrated and/or described communication between the monitoring device 106 and the treating device 110 can be relayed by at least one intermediary device.

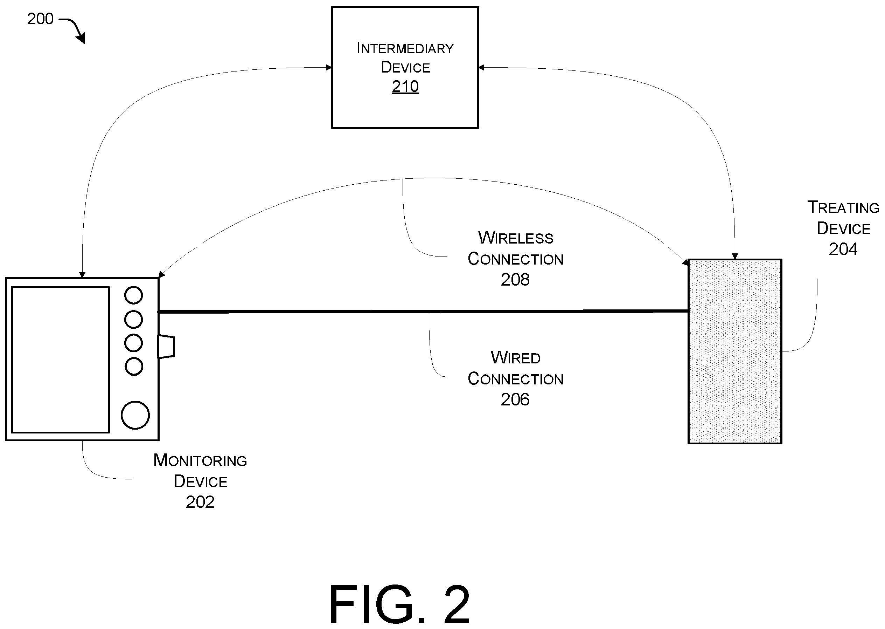

FIG. 2 illustrates an example environment 200 in which a monitoring device 202 and a treating device 204 are configured to communicate via one or more connections. In some examples, the monitoring device 202 is the monitoring device 106 described above with reference to FIGS. 1A and 1B, and the treating device 204 is the treating device 110 described above with reference to FIGS. 1A and 1B.

In some implementations, the monitoring device 202 and the treating device 204 are configured to exchange data over a wired connection 206. In some cases, the wired connection 206 is a physical cable connected to the monitoring device 202 and the treating device 204. The wired connection 206, for instance, is removably coupled to the monitoring device 202 and/or the treating device 204. For instance, a user may physically couple the wired connection 206 to a socket of the monitoring device 202 and/or a socket of the treating device 204. In various cases, data is transmitted over the wired connection 206 via electrical and/or optical signals. Examples of sockets include Universal Service Bus (USB) sockets, micro-USB sockets, sockets specific to medical connectors, and the like.

According to some implementations, the wired connection 206 supports reliable and low-latency communication between the monitoring device 202 and the treating device 204, but may physically impede movements of a rescuer operating the monitoring device 202 and/or treating device 204, movements of a patient being monitored and/or treated by the monitoring device 202 and/or the treating device 204, or even movements of the monitoring device 202 and/or the treating device 204 themselves. In some cases, the wired connection 206 connects the monitoring device 202 and the treating device 204 temporarily during the treatment of a patient at a rescue scene. For instance, the monitoring device 202 and the treating device 204 may exchange data that enables the monitoring device 202 and the treating device 204 to pair and exchange substantive data over an alternate communication channel.

In some cases, the monitoring device 202 and the treating device 204 are configured to exchange data over a wireless connection 208. Examples of the wireless connection 208 include an ultrasonic connection, a Li-Fi connection, an IEEE connection (e.g., a WI-FI, BLUETOOTH, ZIGBEE, etc.), a 3GPP connection (e.g., LTE, NR, etc.), a Global System for Mobile Communications Association (GSMA) connection, a European Telecommunications Standards Institute (ETSI) connection, or the like. In various instances, data is transmitted over the wireless connection 208 via electromagnetic (e.g., RF signaling, visible light, etc.) and/or ultrasonic signals. In some examples, one or more base stations and/or APs facilitate the wireless connection 208 between the monitoring device 202 and the treating device 204.

In various cases, data transmitted over the wireless connection 208 is encrypted. For example, the monitoring device 202 encrypts data, the monitoring device 202 transmits the encrypted data over the wireless connection 208 (e.g., as a wireless signal), and the treating device 204 restores the data by decrypting the encrypted data. Similarly, in some cases, the treating device 204 encrypts data, the treating device 204 transmits the encrypted data over the wireless connection 208 (e.g., as a wireless signal), and the monitoring device 202 restores the data by decrypting the encrypted data. In some cases, the encrypted data includes substantive data, such as physiological parameters, reports, and instructions. In some implementations, the monitoring device 202 and treating device 204 perform encryption and/or decryption using one or more keys. The key(s), for instance, is exchanged during pairing.

According to various implementations, the monitoring device 202 and the treating device 204 are configured to exchange data via an intermediary device 210. The intermediary device 210, for example, includes a passkey, a badge, a card, a mobile device, a tablet computer, an IoT device, a computing device, a server, a hub device, a smart cot device, a smart crash cart, another medical device, or any combination thereof. As used herein, the term "passkey," and its equivalents, may refer to a passive device including an antenna that is powered by an electromagnetic field generated by a separate, active device. That is, a passkey may be powered by inductive coupling. In some cases, when powered, the passkey may act as a transponder by emitting an electromagnetic signal that can be detected by the active device. For instance, the passkey may be a passive NFC target device. In some implementations, the passkey is an ID badge of a rescuer operating the monitoring device 202 and/or the treating device 204.

In some examples, the intermediary device 210 includes or is integrated into a cot. As used herein, the term "cot," and its equivalents, may refer to a portable patient support apparatus. A cot, for instance, may be utilized by one or more rescuers to move a patient from one location to another location. In particular examples, the cot includes a cushioned bed surface that supports the patient in a supine position. The cot, in addition, may include an adjustable frame configured to change and/or support an angle or height at which the bed is disposed. In some cases, the cot further includes retention straps configured to bind the patient to the bed. In some cases, the frame is mechanically coupled to a support that is configured to hold the monitoring device 202 and/or the treating device 204. In addition, the cot may include one or more processors and/or transceivers configured to perform various functions described herein.

In some cases, the monitoring device 210 includes or is integrated into a hub device. As used herein, the terms "hub," and "hub device," and their equivalents, may refer to a device configured to connect and exchange data with multiple external devices. For instance, a hub device may act as an AP or base station connected to multiple devices. The hub device, for example, is portable. In some cases, the hub device is integrated with a transportation device, such as an ambulance, that is configured to move between different locations. The hub device, for instance, may include one or more processors and/or transceivers configured to perform various functions described herein.

The intermediary device 210 may be connected to the monitoring device 202 by a first wired and/or wireless connection, and may be connected to the treating device 204 by a second wired and/or wireless connection. In some implementations, at least one wireless connection that connects the intermediary device 210 to the monitoring device 202, or the intermediary device 210 to the treating device 204, is of a different protocol than the wireless connection 208. For example, the intermediary device 210 may exchange data with the monitoring device 202 and/or the treating device 204 via NFC signaling, whereas data transmitted over the wireless connection 208 may be in the form of RF signaling (e.g., BLUETOOTH signaling).

In various implementations, the intermediary device 210 facilitates pairing between the monitoring device 202 and the treating device 204. For instance, once paired, the monitoring device 202 and the treating device 204 establish and/or exchange data over the wireless connection 208. Further, once paired, the monitoring device or the treating device 204 may rely on parameters reported by the other device, or may perform actions instructed by the other device.

In some implementations, the intermediary device 210 transmits a key to the monitoring device 202 and/or the treating device 204. The monitoring device 202 and/or the treating device 204 use the key to encrypt and/or decrypt data transmitted over the wireless connection 208. In some cases, the intermediary device 210 receives the key from the monitoring device 202 and/or the treating device 204. According to some examples, the intermediary device 210 generates the key. In some examples, the intermediary device 210 provides multiple keys to the monitoring device 202 and/or the treating device 204 for ongoing communication between the monitoring device 202 and the treating device 204.

According to some implementations, the intermediary device 210 pairs with the monitoring device 202 and/or the treating device 204. For instance, the intermediary device 210 may receive a signal from the monitoring device 202 that advertises an identity of the monitoring device 202 (e.g., a numerical identifier associated with the monitoring device 202). A user may operate the intermediary device 210 (for instance, a mobile phone) to select the monitoring device 202 from a drop-down menu output on a touchscreen of the intermediary device 210. In response to the selection, the monitoring device 202 and the intermediary device 210 pair. In some cases, the intermediary device 210 further receives a signal indicating an identity of the treating device 202. The user, for instance, operates the intermediary device 210 to select the treating device 204. In various implementations, the intermediary device 210 transmits data to the monitoring device 202 and/or the treating device 204 that causes pairing between the monitoring device 202 and the treating device 204.

In some examples, the intermediary device 210 in the form of a smart crash cart includes a frame to support and/or hold the monitoring device 202 and/or the treating device 204, and, in some cases, the intermediary device 210 may charge the respective batteries of the devices 202 and 204 to extend the life of the devices 202 and 204 in the field. In some examples, data 114 is shared between the devices 202 and 204 via the intermediary device 210. In some examples, the intermediary device 210 includes a control panel that allows an operator to control one or both of the devices 202 and 204, such as to administer therapy to a patient.

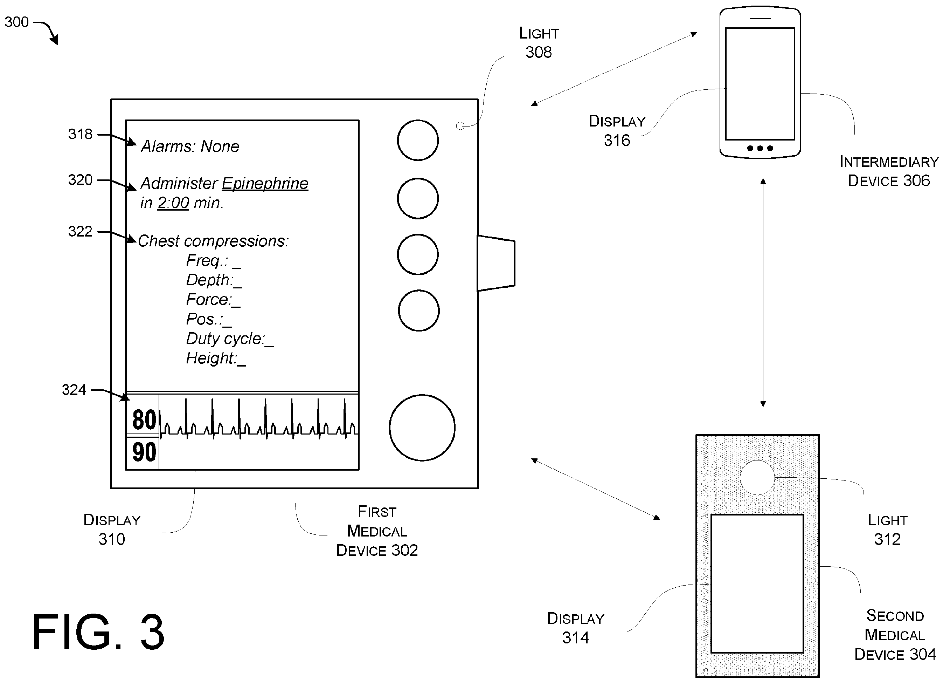

FIG. 3 illustrates example information output via an output device of a medical device based on communication with another medical device. In particular, an environment 300 depicted in FIG. 3 includes a first medical device 302, a second medical device 304, and an intermediary device 306. The first medical device 302 and second medical device 304 may each be any medical device configured to monitor and/or treat an individual. For example, the first medical device 302 can be the same as or similar to the monitor device 106 introduced in FIGS. 1A and 1B, and the second medical device 304 can be the same as or similar to the treating device 110 introduced in FIGS. 1A and 1B. In some cases, the intermediary device 306 is a third medical device (e.g., a smart cot, a smart crash cart, a medication-administering device, etc.) or a non-medical device, such as a general purpose computer, mobile device (e.g., mobile phone), tablet computer, IoT device, hub, base station, AP, or the like.

The first medical device 302 includes one or more output devices configured to output information, which may be consumable by a user in the vicinity of the first medical device 302 or otherwise operating the first medical device 302, such as the rescuer 102 of FIGS. 1A and 1B. The information may also, or alternatively, be consumable by the patient 104 who is receiving treatment administered by the first medical device 302 and/or the second medical device 304. The output devices of the first medical device 302 can include a light(s) 308, and/or a display(s) 310. Although not specifically illustrated, the output devices of the first medical device 302 can further include a transceiver(s) (e.g., a transmitter(s)), a speaker(s), a haptic actuator(s), or any other suitable type of output device(s). In some examples, the second medical device 304 and/or the intermediary device 306 each include some or all of these same types of output devices. For example, FIG. 3 depicts the second medical device 304 as including a light(s) 312 and a display(s) 312, and the intermediary device 306 as including a display(s) 316. Each of the devices 302, 304, and 306 is configured to output information via a respective output device(s) using the various techniques described herein.

The first medical device 302 and the second medical device 304 are also configured to communicate with one another, such as by exchanging coordinated care data 114, as described herein. In some examples, the recipient device of the data 114 and/or the intermediary device 306 is configured to output information via a local output device(s) based on an analysis of the data 114 received by the recipient device. FIG. 3 illustrates various examples of types of information that the first medical device 302 can output on the display 310.

In some examples, the first medical device 302 is configured to output an indication 318 of an alarm (or a lack thereof). For example, the first medical device 302 may receive data 114A from the second medical device 304, determine, by analyzing the data 114A, an alarm associated with the second medical device 304, and output, via an output device(s) (e.g., the display 310), an indication 318 of the alarm. In the example of FIG. 3, the alarm indication 318 indicates that there are no alarms at the present moment. In the case of no alarms, it is to be appreciated that the indication 318 may not be output, in some examples. In some examples, the alarms of the first medical device 302 and the second medical device 304 are synchronized in the sense that one of the medical devices 302 or 304 is configured to output alarms associated with either or both of the medical devices 302 and 304. In the example of FIG. 3, the first medical device 302 may be configured to output alarms associated with either the first medical device 302 or the second medical device 304, or both devices. The indication 318 may indicate various types of alarms. For example, if the data 114A received by the first medical device 302 from the second medical device 304 indicates that there is a malfunction with the operation of the second medical device 304 (e.g., the compressor of the second medical device 304 is not operating properly, is jammed, misaligned, etc.), the alarm indication 318 may indicate such a malfunction associated with the second medical device 304. As another example, the data 114A received by the first medical device 302 from the second medical device 304 may indicate that the remaining battery of the second medical device 304 is below a threshold level, and, by analyzing the data 114A, the first medical device 302 may output, via an output device(s) (e.g., the display 310) an indication 318 of a low battery status of the second medical device 304 (e.g., a low battery alarm). Other examples of alarm indications 318 include an indication 318 that wireless communication (e.g., the communication channel 112) has been lost between the first medical device 302 and the second medical device 304, an indication 318 that wireless communication (e.g., the communication channel 112) has been restored between the first medical device 302 and the second medical device 304, an indication 318 that corrupt data has been received by the first medical device 302 from the second medical device 304, an indication 318 that one or more physiological parameters of the patient 104 monitored by the second medical device 304 are out-of-range (e.g., above or below a threshold value), an indication 318 that the software installed on the second medical device 304 is outdated and/or that a software update is available for the second medical device 304, an indication 318 that motion of the patient 104 has been detected by the second medical device 304, an indication 318 that a device has been removed from the patient 104 (e.g., based on a detection of a change of impedance associated with the patient 104), an indication 318 that the second medical device 304 experienced a self-test error during startup, an indication 318 that the second medical device 304 is performing a self-test at startup, an indication 318 that a compression pattern of chest compressions being administered by the second medical device 304 is too deep (e.g., exceeding a threshold depth), an indication 318 that a compression pattern of chest compressions being administered by the second medical device 304 is too shallow (e.g., below a threshold depth), an indication 318 that a timing failure has occurred with the compression pattern of chest compressions being administered by the second medical device 304, an indication 318 that a temperature of the second medical device 304 is too high (e.g., exceeding a threshold temperature), an indication 318 of an internal hardware error of the second medical device 304, an indication 318 that the temperature of the battery of the second medical device 304 is too high (e.g., exceeding a threshold temperature), an indication 318 that a certain amount of time remains until battery power of the second medical device 304 is depleted, an indication 318 that the compressor of the second medical device 304 is moving to a retracted (e.g., upper) position while the second medical device 304 is powering off, an indication 318 that the compressor of the second medical device 304 is below a lowest start position (indicating that the patient 104 is too small to be treated by the second medical device 304), an indication 318 that a gap between a pressure pad of the compressor of the second medical device 304 and the patient's chest is detected, an indication 318 that the compressor of the second medical device 304 is pressed down when the second medical device 304 is locked in a "pause" mode, an indication 318 associated with a ventilation (e.g., an upcoming ventilation, ventilation pause, etc.), an indication 318 that an internal temperature of the second medical device 304 is rising, and/or an indication 318 associated with CPR or continuous timers prompting the operator for action. Outputting such alarm indications 318 via an output device(s) of the first medical device 302 may be beneficial if, say, the second medical device 304 does not include an output device, or if an operator (e.g., a rescuer 102) is in a better position to consume (e.g., view) information output via an output device of the first medical device 302 notwithstanding an ability of the second medical device 304 to output the same or similar alarm indication 318.

In some examples, the first medical device 302 is configured to output an indication 320 of a medication to administer the patient 104. In an example where the second medical device 304 is a mechanical chest compression device administering chest compressions (e.g., CPR therapy) to the patient 104, the medication associated with indication 320 may be a medication to administer to the patient 104 during CPR, such as epinephrine, amiodarone, or the like. In some examples, the first medical device 302 and/or the second medical device 304 is configured to reference guidelines and/or utilize an algorithm to determine what type of medication to administer and when to administer the medication to the patient 104 undergoing CPR. For example, guidelines and/or an algorithm may cause the first medical device 302 to output an indication 320 that a particular medication should be administered to the patient 104 at timely intervals, such as every 2, 4, or 6 minutes. In the example of FIG. 3, the medication indication 320 indicates that epinephrine should be administered to the patient 104 in 2 minutes. The time of "2 minutes" in the example indication 320 may be a countdown timer that decrements by seconds, minutes, etc. as time passes, and the alarm indication 318 may alert an operator (e.g., the rescuer 102) that the medication should be administered when the countdown timer reaches zero. In some examples, the medication indication 320 output by the first medical device 302 is based on data obtained by the first medical device 302, such as via the sensor 108 described above. For example, the first medical device 302 may represent a monitor-defibrillator that is configured to monitor physiological parameters including blood pressure, blood oxygenation, an end tidal gas parameter (e.g., ETCO₂), or the like, and by analyzing such physiological parameter data, the first medical device 302 may determine a medication to administer to the patient 104, and, in some examples, a time at which to administer the medication. Accordingly, the indication 320 may indicate the medication and the time at which to administer the medication. In some examples, the first medical device 302 may receive data 114A from the second medical device 304, determine, by analyzing the data 114A, a medication to administer the patient 104, and output, via an output device(s) (e.g., the display 310), an indication 320 of the medication. For instance, based on a parameter(s) of the chest compressions being administered to the patient 104 by the second medical device 304, the first medical device 302 may determine a type of medication to administer to the patient 104 and a time at which to administer the medication. Accordingly, the indication 320 may indicate the medication and the time at which to administer the medication. In some examples, the intermediary device 306 represents a device that is configured to administer medications automatically, without user intervention, such as via an IV used to administer medications intravenously. In this example, the medication and the time at which to administer the medication, as determined by the first medical device 302, may be communicated to the intermediary device 306 to automatically administer the medication(s) to the subject 104 at the appropriate times and/or display the indication 320 on the display 316 of the intermediary device 306. Thereto, the intermediary device 306 may generate an internal control signal to automatically administer the medication(s) to the subject 104 at the appropriate times, based on the data received from the first medical device 302.

In some examples, the first medical device 302 is configured to output an indication 322 of a treatment parameter(s) associated with therapy (e.g., chest compressions) being administered to the patient 104 by the second medical device 304. For example, the first medical device 302 may receive data 114A from the second medical device 304, determine, by analyzing the data 114A, a treatment parameter(s) associated with chest compressions that are being administered to the patient 104, such as a frequency, timing, duty cycle, depth, force, velocity, acceleration, height, and/or position of the chest compressions. The example of FIG. 3 shows these treatment parameters output via the indication 322 without any values, which may be the case before the second medical device 304 starts administering therapy (e.g., chest compressions) to the patient 104. However, once the second medical device 304 starts to administer therapy (e.g., chest compressions) to the patient 104, the second medical device 304 may send data 114A indicative of these treatment parameters to the first medical device 302, and upon analyzing the data 114A, the first medical device may determine the treatment parameter(s) associated with the second medical device 304, and output, via an output device(s) (e.g., the display 310) the indication 322 of the frequency, timing, duty cycle, depth, force, velocity, acceleration, height, and/or position of the chest compressions being administered to the patent 104.

In some examples, an operator of the first medical device 302 can operate the first medical device 302 to manually adjust one or more of the treatment parameters associated with therapy (e.g., chest compressions) being administered to the patient 104 by the second medical device 304. For example, the operator may provide user input to the display 310 to select a treatment parameter (e.g., the depth parameter) associated with the second medical device 304, and, in response to selecting the treatment parameter, an interactive element(s) (e.g., an up arrow and a down arrow, plus and minus icons, etc.) may be displayed on the display 310 that, when selected, causes the treatment parameter to be adjusted to a new value. In response to the operator adjusting a treatment parameter, the first medical device 302 can send data 114B to the second medical device 304, the second data 114B including a control parameter and/or the adjusted treatment parameter to adjust the treatment parameter (e.g., the depth parameter) to the new value. The second medical device 304 may analyze the data 114B to determine the new value for the treatment parameter, and may adjust the treatment parameter to the new value. In this way, an operator (e.g., the rescuer 102) can remotely adjust treatment parameters relating to the therapy that is being administered by the second medical device 304, such as by providing user input to the first medical device 302 (e.g., via a touch screen display 310, buttons, knobs, voice commands, etc.). It is to be appreciated that other control operations may be performed manually by an operator providing user input to the first medical device 302, such as pausing the therapy that is being administered by the second medical device 304, resuming the therapy that is being administered by the second medical device 304, or the like.

In some examples, the first medical device 302 is configured to output an indication 324 of a physiological parameter(s), such as an ECG (e.g., ECG waveform), associated with the patient 104. Because the second medical device 304 may be administering therapy (e.g., chest compressions), the physiological monitoring and analysis (e.g., ECG analysis) performed by the first medical device 302 may be coordinated with one or more pauses or intervals in the therapy (e.g., pauses or intervals between sequential chest compressions) to optimize the resultant signal and/or to minimize interruptions to the therapy (e.g., chest compressions) being administered by the second medical device 304. In some examples, a chest compression artifact may be removed by the first medical device 302 in order to optimize physiological parameter monitoring and analysis (e.g., ECG analysis). For example, the first medical device 302 may be configured to determine a timing and/or frequency of the chest compressions being administered to the patient 104 by the second medical device 304 in various ways, such as by using the sensor 108 and/or by receiving data 114A from the second medical device 304. To illustrate, the sensor 108 (introduced in FIGS. 1A and 1B) may include an ECG sensor (e.g., multiple electrodes, such as ECG electrodes, disposed on, or affixed to, the chest of the patient 104) that is configured to detect a signal that includes the ECG of the patient 104. The first medical device 302 includes a detection circuit configured to detect a voltage between the ECG electrodes over time. The voltage over time, for instance, includes the ECG of the patient 104. In various implementations, the signal detected by the sensor 108 also includes an artifact that is present due to chest compressions being administered to the patient 104 by the second medical device 304. The first medical device 302 is configured to analyze the signal detected by the ECG sensor. In various cases, the first medical device 302 identifies the timing (e.g., identifies the artifact in the voltage over time) and/or frequency of the chest compressions administered to the patient 104 (e.g., by determining the frequency at which the artifact appears in the voltage over time). In another example, the first medical device 302 may receive data 114A from the second medical device 304, determine, by analyzing the data 114A, a timing and/or a frequency of the chest compressions being administered to the patient 104. In either case, the first medical device 302 is configured to identify, select, or generate a filter (e.g., a digital filter) characterized by the timing and/or the frequency of the chest compressions that are being administered by the second medical device 304. For instance, the first medical device 302 identifies, selects, or generates a comb filter that rejects a band corresponding to the chest compression frequency as well as one or more harmonics of the chest compression frequency. The first medical device 302 removes the chest compression artifact from the physiological data (e.g., ECG) representing a physiological parameter of the patient 104 by applying the filter (e.g., the digital filter) to the physiological data (e.g., by applying the filter to the voltage over time detected by the sensor 108). The patient's 104 physiological parameter(s) (e.g., ECG) is thereby optimized (e.g., more pronounced) in the resultant signal than the signal in which the chest compression artifact is present.

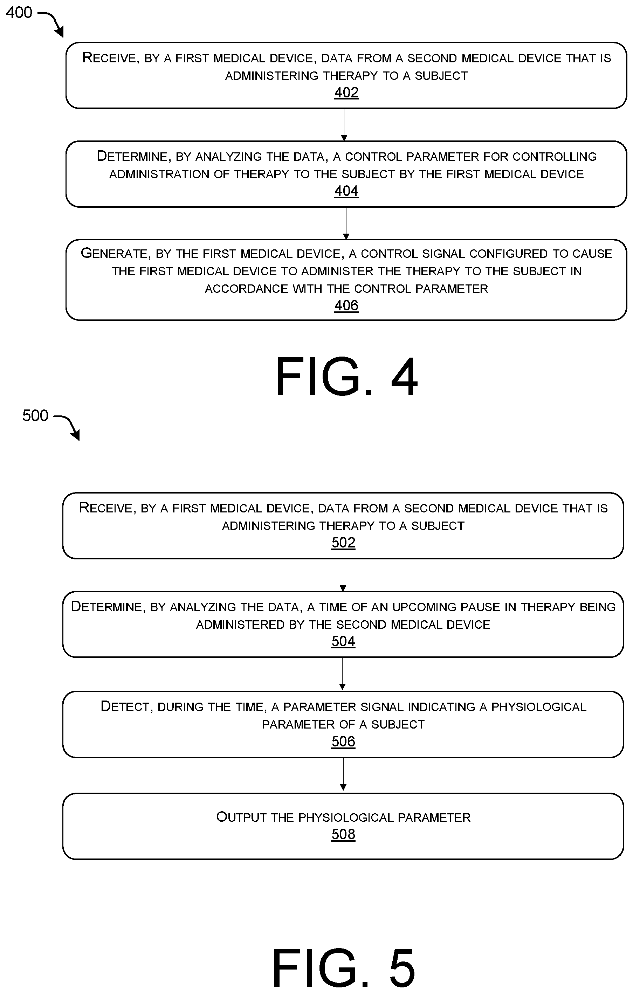

FIGS. 4, 5, 6, and 7 illustrate processes performed by one or more systems, devices, or entities described herein. For example, the processes illustrated in FIGS 4, 5, 6, and 7 are implemented by an entity including at least one of the monitoring device 106, the monitoring device 202, the first medical device 302, the treating device 110, the treating device 204, the second medical device 304, any type of medical device described herein, or at least one processor configured to execute instructions.

FIG. 4 illustrates an example process 400 for assisting in administering therapy by a first medical device based on data received from a second medical device. At 402, a first medical device receives data from a second medical device that is administering therapy to a subject (e.g., a patient 104). In some implementations, the data received at block 402 represents the coordinated care data 114 described herein. This coordinated care data 114 represents substantive data relevant to monitoring and/or treating the subject. In some implementations, the data 114 received at block 402 indicates one or more physiological parameters of the subject, such as an ECG of the subject, an intrinsic heartbeat of the subject, or any other suitable physiological parameter described herein. In some implementations, the data 114 received at block 402 includes a treatment parameter associated with a treatment (e.g., therapy) that is being administered by the second medical device. In some implementations, the data 114 received at block 402 includes one or more instructions, such as an instruction for the first medical device to detect a physiological parameter of the subject, to administer a treatment to the subject, to cease administering a treatment to the subject, to modify a treatment of the subject, or any combination thereof. In some implementations, the data 114 received at block 402 includes synchronization data that enables the first medical device and the second medical device to synchronize clocks.

At 404, the first medical device determines, by analyzing the data 114, a control parameter(s) for controlling administration of a therapy to the subject by the first medical device. In some implementations, the control parameter(s) determined at block 404 is a timing parameter indicative of a timing with which the therapy is to be administered. Such a timing parameter may be a frequency at which to administer the therapy, a duty cycle with which to administer the therapy, or the like. In some implementations, the control parameter(s) determined at block 404 is an intensity parameter indicative of an intensity level or an extent to which the therapy is to be performed (e.g., depth, height, force, etc.). In some implementations, the control parameter(s) determined at block 404 is a position parameter, such as a position with respect to the subject (e.g., a position on the subject's body, such as the chest of the subject) at which the therapy is to be performed.

The first medical device generates a control signal configured to cause the first medical device to administering the therapy to the subject in accordance with the control parameter. Thus, the process 400 is only concerned with the operating of a the first medical device without any functional link between the process and the effects produced by the first medical device on the body. The process 400 excludes the step 406 of administering the therapy to the subject in accordance with the control parameter. For example, the process 400 assists in allowing the first medical device to administer the therapy at block 406 in accordance with a timing, intensity, extent, and/or position determined at block 404. To illustrate how the process 400 may be implemented, example use cases will now be described.

In a particular example implementation of the process 400, the first medical device is a monitor-defibrillator (e.g., an external defibrillator) and the second medical device is a mechanical chest compression device. In this particular example, at 402, the monitor-defibrillator receives (e.g., via a transceiver) data 114A from the mechanical chest compression device that is administering chest compressions to a subject. At 404, the monitor-defibrillator determines, by analyzing the data 114A, a control parameter(s) for controlling administration of therapy 116 to the subject by the monitor-defibrillator. At 406, the monitor-defibrillator generates a control signal configured to cause the monitor-defibrillator to administer the therapy 116 to the subject in accordance with the control parameter(s). In some implementations, the control signal is configured for causing a discharge circuit of the monitor-defibrillator to output an electrical shock to the subject. In some implementations, the control signal configured to cause defibrillation. In some implementations, the control signal is configured to cause pacing. In some implementations, the control parameter determined at block 404 is indicative of a timing with which the therapy 116 is to be administered to the subject by the monitor-defibrillator in coordination with chest compressions that are being administered to the subject by the mechanical chest compression device. In this example, the control signal is configured to cause the therapy 116 to be administered (e.g., an electrical shock is delivered) to the subject by the monitor-defibrillator in accordance with the timing. This allows for coordinating the respective therapies (e.g., electrical shocks and chest compressions) that are to be administered to the subject by the monitor-defibrillator and the mechanical chest compression device. In some implementations, the control parameter determined at block 404 is a shock parameter indicative of a level at which an electrical shock is to be delivered to the subject by the monitor-defibrillator. In this example, the control signal is configured to cause an electrical shock to be delivered at the shock level to the subject by the monitor-defibrillator.

In another example implementation of the process 400, the first medical device is a mechanical chest compression device and the second medical device is a monitor-defibrillator (e.g., an external defibrillator). In this particular example, at 402, the mechanical chest compression device receives (e.g., via a transceiver) data 114B from the monitor-defibrillator that is administering therapy (e.g., defibrillation, pacing, etc.) to a subject. At 404, the mechanical chest compression device determines, by analyzing the data 114B, a control parameter(s) for controlling administration of therapy 118 (e.g., chest compressions) to the subject by the mechanical chest compression device. At 406, the mechanical chest compression device generates a control signal configured to cause the mechanical chest compression device to administer the therapy 116 to the subject in accordance with the control parameter(s). In some implementations, the control parameter determined at block 404 is a frequency at which to administer chest compressions to the subject. In this example, the control signal is configured to cause the chest compressions to be administered to the subject at the determined frequency. In some implementations, the data 114B is indicative of an intrinsic heartbeat of the subject, and the frequency of the chest compressions to be administered is coordinated with the intrinsic heartbeat of the subject. In some implementations, the control parameter determined at block 404 is a position with respect to the subject at which the chest compressions are to be administered. In this example, the control signal is configured to cause the chest compressions to be administered to the subject at the determined position. In some implementations, the control parameter determined at block 404 is a depth to which the chest compressions are to be administered to the subject. In this example, the control signal is configured to cause the chest compressions to be administered to the subject to the determined depth. In some implementations, the control parameter determined at block 404 is a force with which the chest compressions are to be administered to the subject. In this example, the control signal is configured to cause the chest compressions to be administered to the subject with the determined force. In some implementations, a combination of these example control parameters and/or other example control parameters described herein may be determined and used to generate the control signal configured to control the administration of the chest compressions. As indicated above, the data 114B that is analyzed to determine the control parameter(s) can be physiological parameter data detected by the monitor-defibrillator, such as via the sensor 108 described herein. Example physiological parameters included in such physiological parameter data include ETCO₂ of the subject (e.g., a reduction in the ETCO₂ sensed in the airway of the subject may indicate reduced pulmonary blood flow, which may take into account any recent administration of epinephrine to the subject, which may constrict peripheral blood vessels and reduce overall forward blood flow through the lungs, and, therefore, carbon dioxide), a photoplethysmography signal (e.g., indicating a decreased amplitude of the photoplethysmography signal gathered by a pulse oximeter module of the monitor-defibrillator, taking into account any recent administration of epinephrine to the subject), blood pressure of the subject (e.g., invasive blood pressure detected by the monitor-defibrillator using an intraarterial line and/or sensor in an artery, which may indicate a decrease in systolic or mean blood pressure, or possibly a decrease in diastolic blood pressure produced by ongoing CPR), cerebral oxygen saturation (SrO₂) of the subject, ECG of the subject, ultrasound images and/or signals of the subject (e.g., doppler indicating that the flow in a vessel has decreased to a value below a threshold, imaging that suggests the mechanical chest compression device is compressing the outflow tract of the left ventricle, etc.), or the like. Accordingly, if the physiological parameter data indicates that the subject is not responding well to the ongoing chest compressions, a control parameter(s) of the chest compressions can be adjusted on-the-fly to improve the outcome of the subject. In some implementations, the control parameter(s) may cause the mechanical chest compression device to adjust itself, such as by moving with respect to the subject (e.g., to a different position on the subject's chest). The mechanical chest compression device may include a motor(s) and gear assembly to adjust the position of a particular component, such as a compressor. In a particular example, the ECG (e.g., ECG waveform) of the subject monitored by the monitor-defibrillator may be indicative of a treatment parameter(s) (e.g., frequency, force, depth, position, etc.) of the chest compressions that is/are being administered to the subject by the mechanical chest compression device, and if the treatment parameter(s) indicates that the compressor of the mechanical chest compression device is misaligned with respect to the subject and/or that the mechanical chest compression device is administering chest compressions to a suboptimal depth (e.g., too shallow or too deep), the mechanical chest compression device may adjust itself by moving (e.g., moving the compressor) with respect to the subject to improve the alignment between the compressor and the subject, and/or by adjusting the depth to which the chest compressions are to be administered to the subject, etc.

FIG. 5 illustrates an example process 500 for coordinating the detection of a physiological parameter of a subject by a first medical device during a pause in therapy that a second medical device is administering to the subject. At 502, a first medical device receives data from a second medical device that is administering therapy to a subject (e.g., a patient 104). The data (e.g., coordinated care data 114) received at block 502 may be the same or similar data to that described above with respect to block 402 of the process 400.

At 504, the first medical device determines, by analyzing the data 114, a time of an upcoming pause or interval in the therapy being administered to the subject by the second medical device. For example, if the data the data 114 received at block 402 includes a treatment parameter associated with a treatment (e.g., therapy) that is being administered by the second medical device, the first medical device may determine, by analyzing the data, the timing and/or frequency of ongoing therapy that is being administered by the second medical device to determine (e.g., predict) a time at which an upcoming pause or interval in the therapy will occur.

At 506, the first medical device detects, during the time of the pause or interval determined at block 504, a parameter signal indicating a physiological parameter of the subject. In other words, the first medical device waits for the pause or interval in the therapy being administered by the second medical device to detect the parameter signal.

At 508, the first medical device optionally outputs the physiological parameter and/or information relating thereto via an output device(s) (e.g., a display) of the first medical device. To illustrate how the process 500 may be implemented, example use cases will now be described.

In a particular example implementation of the process 500, the first medical device is a monitor-defibrillator (e.g., an external defibrillator) and the second medical device is a mechanical chest compression device. In this particular example, at 502, the monitor-defibrillator receives (e.g., via a transceiver) data 114A from the mechanical chest compression device that is administering chest compressions to a subject. At 504, the monitor-defibrillator determines, by analyzing the data 114A, a time of an upcoming pause that will follow one of the chest compressions administered to the subject by the mechanical chest compression device. At 506, the monitor-defibrillator detects a physiological parameter (e.g., an ECG) of the subject during the time of the pause determined at block 504. At 508, the monitor-defibrillator optionally outputs the physiological parameter (e.g., the ECG, such as an ECG waveform) via an output device(s) (e.g., a display(s)) of the monitor-defibrillator. This can optimize the monitoring of the subject's physiology while minimizing interruptions to, and/or the downtime of, the mechanical chest compression device so that chest compressions can continue without interruption while the monitor-defibrillator monitors the physiological parameter of the subject.

In another example implementation of the process 500, the first medical device is a mechanical chest compression device and the second medical device is a monitor-defibrillator (e.g., an external defibrillator). In this particular example, at 502, the mechanical chest compression device receives (e.g., via a transceiver) data 114B from the monitor-defibrillator that is administering therapy (e.g., pacing therapy) to a subject. At 504, the mechanical chest compression device determines, by analyzing the data 114B, a time of an upcoming pause that will follow one of the electrical shocks delivered to the subject by the monitor-defibrillator. At 506, the mechanical chest compression device detects a physiological parameter of the subject during the time of the pause determined at block 504. At 508, the mechanical chest compression device optionally outputs the physiological parameter via an output device(s) (e.g., a display(s)) of the mechanical chest compression device. This can optimize the monitoring of the subject's physiology while minimizing interruptions to, and/or the downtime of, the monitor-defibrillator so that therapy (e.g., pacing therapy) can continue without interruption while the mechanical chest compression device monitors the physiological parameter of the subject.

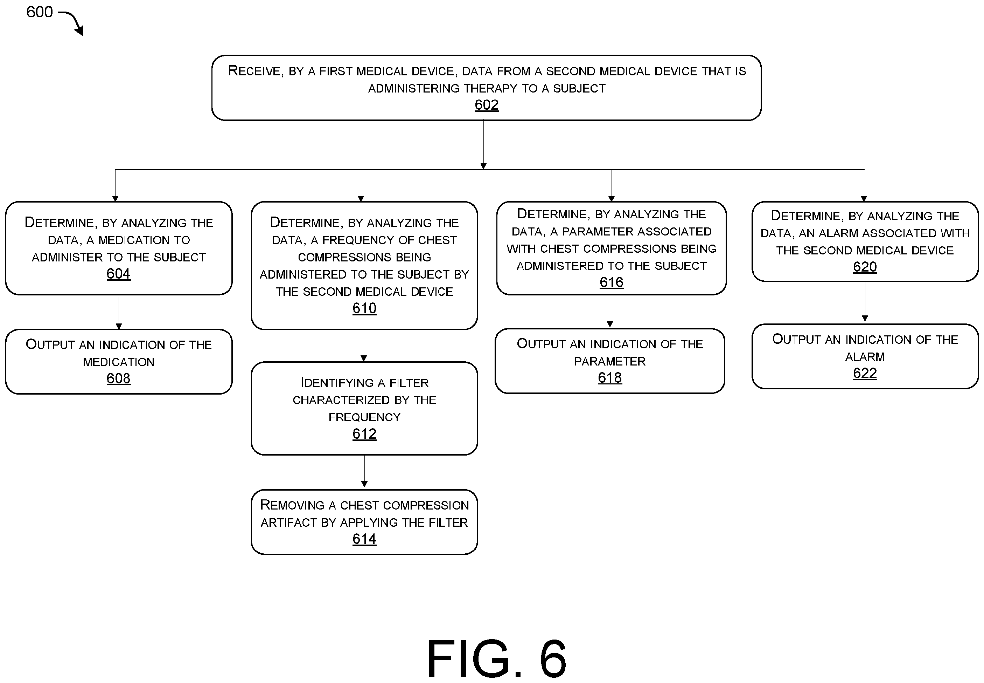

FIG. 6 illustrates an example process 600 for outputting information via an output device of a first medical device by analyzing data received from a second medical device. At 602, a first medical device receives data from a second medical device that is administering therapy to a subject (e.g., a patient 104). The data (e.g., coordinated care data 114) received at block 602 may be the same or similar data to that described above with respect to block 402 of the process 400.

At 604, the first medical device determines, by analyzing the data 114, a medication to administer the subject. In a particular example implementation of the process 600, the first medical device is a monitor-defibrillator (e.g., an external defibrillator) and the second medical device is a mechanical chest compression device administering chest compressions (e.g., CPR therapy) to the subject. In this particular example, the data 114 analyzed at block 604 may include a treatment parameter(s) of the chest compressions being administered to the subject by the mechanical chest compression device, such as the frequency, timing, duty cycle, depth, force, velocity, acceleration, height, and/or position of the chest compressions being administered to the subject. Accordingly, the monitor-defibrillator may determine a type of medication to administer to the subject and when to administer the medication by analyzing the treatment parameter(s) of the chest compressions.

At 608, the first medical device outputs an indication 320 of the medication via an output device of the first medical device. In some examples, this indication 320 indicates a time at which to administer the medication. An example of this indication 320 is depicted in FIG. 3, above. In some examples, the indication 320 may be output via a transceiver for receipt by an intermediary device 306 that is configured to administer medications to the subject automatically, without user intervention, such as via an IV used to administer medications intravenously. In some examples, the medication associated with indication 320 output at block 608 may be a medication to administer to the patient 104 during CPR, such as epinephrine, amiodarone, or the like. It is also to be appreciated that the determination of the medication may occur without receiving the data 114 at block 602 and without analyzing the data 114 at block 604. For example, as described above with reference to FIG. 3, the first medical device (e.g., monitor-defibrillator) may be configured to reference guidelines and/or utilize an algorithm to determine a type of medication to administer and when to administer the medication to the subject who may be receiving therapy (e.g., undergoing CPR) administered by the second medical device (e.g., mechanical chest compression device).

At 610, the first medical device determines, by analyzing the data 114, a frequency of the chest compressions that are being administered to the subject by the second medical device. For instance, if the first medical device is a monitor-defibrillator, and the second medical device is a mechanical chest compression device, the data 114 received from the mechanical chest compression device at block 602 may indicate one or more treatment parameters of the chest compressions that are being administered, such as a timing and/or frequency of the chest compressions.

At 612, the first medical device identifies, selects, or generates a filter (e.g., a digital filter) characterized by the frequency of the chest compressions. For instance, the first medical device (e.g., monitor-defibrillator) identifies, selects, or generates a comb filter that rejects a band corresponding to the chest compression frequency, and/or one or more harmonics of the chest compression frequency.

At 614, the first medical device removes a chest compression artifact from physiological data representing a physiological parameter of the subject by applying the filter to the physiological data. For example, the first medical device (e.g., monitor-defibrillator) may removes the chest compression artifact from the ECG of the subject by applying the filter (e.g., the digital filter) to the ECG data obtained by the first medical device (e.g., monitor-defibrillator), such as via the sensor 108. In some examples, removing the chest compression artifact at block 614 includes outputting an indication 324 of the physiological parameter with the chest compression artifact removed using the filter. An example of this is depicted in FIG. 3.

At 616, the first medical device determines, by analyzing the data 114, a parameter associated with the chest compressions that are being administered to the subject by the second medical device. For instance, if the first medical device is a monitor-defibrillator, and the second medical device is a mechanical chest compression device, the data 114 received from the mechanical chest compression device at block 602 may indicate one or more treatment parameters of the chest compressions that are being administered, such as the frequency, timing, duty cycle, depth, force, velocity, acceleration, height, and/or position of the chest compressions. For example, by analyzing the data 114, a frequency of the chest compressions may be determined by the first medical device (e.g., monitor-defibrillator) at block 616. As another example, by analyzing the data 114, a depth of the chest compressions may be determined by the first medical device (e.g., monitor-defibrillator) at block 616. As another example, by analyzing the data 114, a force with which the chest compressions are being administered may be determined by the first medical device (e.g., monitor-defibrillator) at block 616. As another example, by analyzing the data 114, a position with respect to the subject at which the chest compressions are being administered may be determined by the first medical device (e.g., monitor-defibrillator) at block 616. These are merely examples of treatment parameters that can be determined at block 616.

At 618, the first medical device outputs an indication 322 of the chest compression parameter via an output device of the first medical device (e.g., monitor-defibrillator). An example of this is depicted in FIG. 3 where the first medical device (e.g., monitor-defibrillator) outputs an indication of the frequency, timing, duty cycle, depth, force, velocity, acceleration, height, and/or position of the chest compressions. This may aid an operator (e.g., a rescuer 102) in assessing the therapy that is being administered by the second medical device (e.g., mechanical chest compression device) if, say, the operator is closer to the first medical device (e.g., monitor-defibrillator) and farther from the second medical device (e.g., mechanical chest compression device).

At 620, the first medical device determines, by analyzing the data 114, an alarm associated with the second medical device. As described above by way of example, the alarm may indicate a malfunction of the second medical device, a low battery of the second medical device, or any other suitable event and/or state of the second medical device that may be cause for alerting an operator (e.g., a rescuer 102) in the vicinity of the subject.

At 622, the first medical device outputs an indication 318 of the alarm via an output device of the first medical device. Although the example of FIG. 3 illustrates an alarm indicator 318 being output via a display, it is to be appreciated that the alarm indication 318 output at block 622 may be output via any additional or different type of output device, such as the light 308 (e.g., by outputting a particular color, such as red, flashing, strobing, etc.), and/or a speaker (e.g., by outputting an alarm type of sound, such as a beeping sound, a siren, etc.). Accordingly, the process 600 may allow for outputting various types of information in a coordinated care scenario involving multiple medical devices, which may assist an operator (e.g., a rescuer 102) to provide improved treatment to the subject.

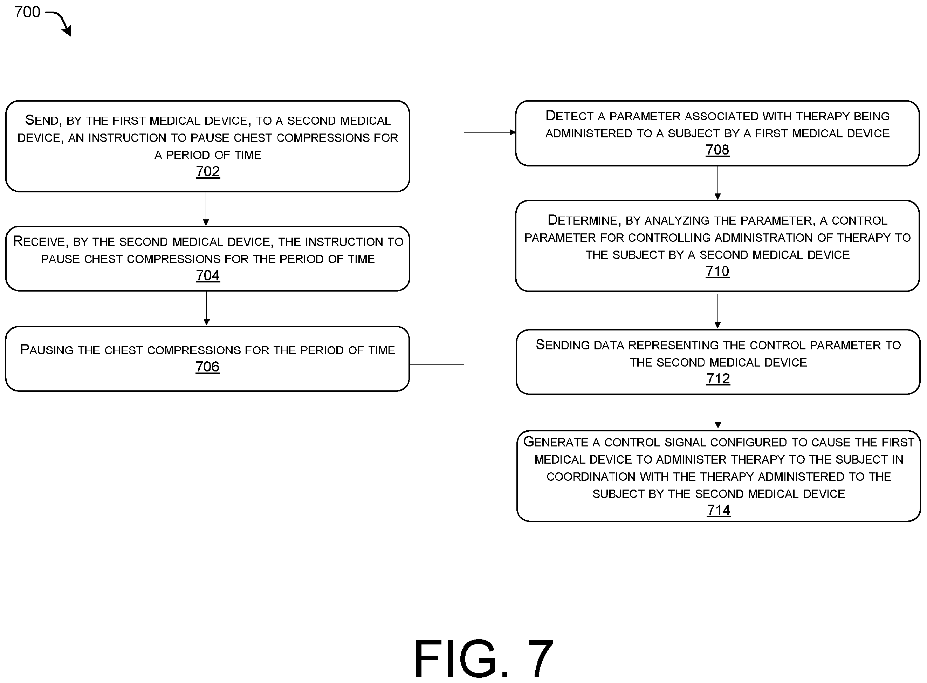

FIG. 7 illustrates an example process 700 for pausing therapy that a second medical device is administering to a subject in order to detect a parameter associated with therapy that is being administered to the subject by a first medical device, and analyzing the parameter to determine how to coordinate therapies administered by the medical devices. At 702, a first medical device (e.g., monitor-defibrillator) sends (e.g., via a transceiver), to a second medical device (e.g., mechanical chest compression device), an instruction to pause (or to cease administering) the chest compressions for a period of time. At 704, the second medical device (e.g., mechanical chest compression device) receives (e.g., via a transceiver), the instruction from the first medical device (e.g., monitor-defibrillator). The instruction can indicate a start time of the pause and a pause duration. Although it may not be ideal to interrupt the ongoing therapy that is being administered by the second medical device (e.g., mechanical chest compression device), the start time of the pause and the pause duration may be selected to optimize detection of a parameter of a patient, which may otherwise be inhibited by, or more difficult to detect, without actively pausing the chest compressions.

At 706, the second medical device (e.g., mechanical chest compression device) pauses the chest compressions for the specific period of time (e.g., the pause duration). In some examples, the second medical device (e.g., mechanical chest compression device) waits for the start time and initiates the pause of the chest compressions for the pause duration at the start time.

At 708, the first medical device detects, during the period of time that the chest compressions are paused, a parameter associated with the therapy that is being administered to the subject by the first medical device. In some implementations, the parameter detected at block 708 is a physiological parameter of the subject, such as any of the physiological parameters described herein. Such physiological parameters may be detected using the sensor 108 described herein. The value of the detected physiological parameter may indicate the health and/or recovery status of the subject, for example, and, in some cases, the detection or measurement of such physiological parameters may be inaccurate if detected during an active chest compression (e.g., a downstroke of a compressor of the mechanical chest compression device). In some implementations, the parameter detected at block 708 is a timing parameter indicative of a timing with which the therapy is being administered to the subject by the first medical device (e.g., monitor-defibrillator), such as a timing of pacing pulses that are being delivered to the subject.

At 710, the first medical device (e.g., monitor-defibrillator) determines, by analyzing the parameter, a control parameter for controlling the administration of therapy to the subject by the second medical device (e.g., mechanical chest compression device). In some implementations, the control parameter determined at block 710 is a frequency, timing, duty cycle, depth, force, velocity, acceleration, height, and/or position of the chest compressions that are to be administered to the subject. For example, by analyzing the parameter, the first medical device (e.g., monitor-defibrillator) may determine, at block 710, a frequency at which to administer chest compressions to the subject, and/or a position with respect to the subject at which chest compressions are to be administered, and/or a depth to which chest compressions are to be administered to the subject, and/or a force with which the chest compressions are to be administered to the subject, etc.

At 712, first medical device (e.g., monitor-defibrillator) sends (e.g., via a transceiver), to a second medical device (e.g., mechanical chest compression device), data 114B representing the control parameter determined at block 710. For example, the data sent to the second medical device (e.g., mechanical chest compression device) may represent a frequency, timing, duty cycle, depth, force, velocity, acceleration, height, and/or position of the chest compressions that are to be administered to the subject.

At 714, the first medical device (e.g., monitor-defibrillator) generates a control signal configured to cause the first medical device to administer therapy (e.g., defibrillation, pacing, etc.) in coordination with the therapy (e.g., chest compressions) administered by the second medical device (e.g., mechanical chest compression device) in accordance with the control parameter. For example, the control parameter sent in the data 114B at block 712 may optimize the frequency of the chest compressions for the delivery of an electrical shock to the subject by the first medical device (e.g., monitor-defibrillator) during an interval between sequential chest compressions. In some implementations, the control signal is configured to cause the therapy to be administered by the first medical device (e.g., monitor-defibrillator) by causing a discharge circuit of the first medical device (e.g., monitor-defibrillator) to output an electrical shock to the subject in coordination with the chest compressions (e.g., in coordination with the frequency of the chest compressions) administered by the second medical device (e.g., mechanical chest compression device) in accordance with the control parameter.

It is to be appreciated that the process 700 may omit blocks 702 to 706, in some examples. In other words, the first medical device (e.g., monitor-defibrillator) may not actively instruct the second medical device (e.g., mechanical chest compression device) to pause the chest compressions before detecting the parameter at block 708. Rather, the first medical device (e.g., monitor-defibrillator) may detect the parameter at block 708 without interrupting the therapy being administered by the second device (e.g., mechanical chest compression device), such as by timing the detection of the parameter for a time that is within an interval between sequential chest compressions, and/or by removing a chest compression artifact using a digital filter, as described herein. Furthermore, the process 700 excludes the step of administering therapy, block 714. Thus, the process 700 is only concerned with the operating of a the first medical device without any functional link between the process and the effects produced by the first medical device on the body. The first medical device may actively control the operation of the second medical device based on a parameter detected by the first medical device through the execution of blocks 708 to 712.

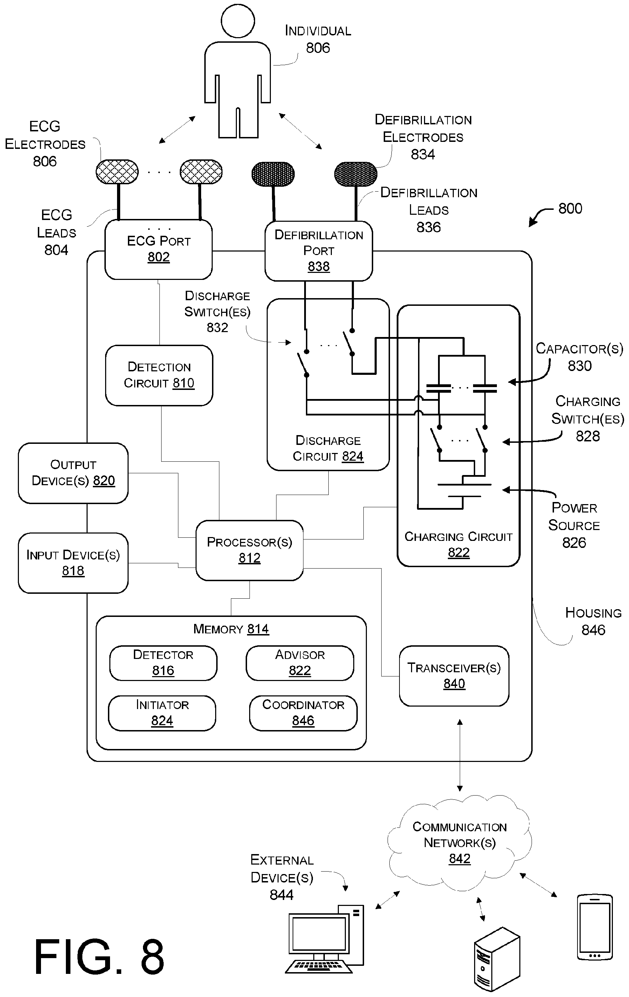

FIG. 8 illustrates an example of an external defibrillator 800 configured to perform various functions described herein. For example, the external defibrillator 800 is the monitoring device 106, the treating device 110, the monitoring device 202, the treating device 204, the first medical device 302, the second medical device 304, the intermediary device 210, or the intermediary device 306 described above with reference to FIGS. 1A to 3.

The external defibrillator 800 includes an electrocardiogram (ECG) port 802 connected, or connectable, to multiple ECG leads 804. In some cases, the ECG leads 804 are removeable from the ECG port 802. For instance, the ECG leads 804 are plugged into the ECG port 802. The ECG leads 804 are connected to ECG electrodes 806, respectively. In various implementations, the ECG electrodes 806 are disposed on different locations on an individual 808. A detection circuit 810 is configured to detect relative voltages between the ECG electrodes 806. These voltages are indicative of the electrical activity of the heart of the individual 808.

In various implementations, the ECG electrodes 806 are in contact with the different locations on the skin of the individual 808. In some examples, a first one of the ECG electrodes 806 is placed on the skin between the heart and right arm of the individual 808, a second one of the ECG electrodes 806 is placed on the skin between the heart and left arm of the individual 808, and a third one of the ECG electrodes 806 is placed on the skin between the heart and a leg (either the left leg or the right leg) of the individual 808. In these examples, the detection circuit 808 is configured to measure the relative voltages between the first, second, and third ECG electrodes 806. Respective pairings of the ECG electrodes 806 are referred to as "leads," and the voltages between the pairs of ECG electrodes 806 are known as "lead voltages." In some examples, more than three ECG electrodes 806 are included, such that 5-lead or 12-lead ECG signals are detected by the detection circuit 810.

The detection circuit 810 includes at least one analog circuit, at least one digital circuit, or a combination thereof. The detection circuit 810 receives the analog electrical signals from the ECG electrodes 806, via the ECG port 802 and the ECG leads 804. In some cases, the detection circuit 810 includes one or more analog filters configured to filter noise and/or artifact from the electrical signals. The detection circuit 810 includes an analog-to-digital (ADC) in various examples. The detection circuit 810 generates a digital signal indicative of the analog electrical signals from the ECG electrodes 806. This digital signal can be referred to as an "ECG signal" or an "ECG."

In some cases, the detection circuit 810 further detects an electrical impedance between at least one pair of the ECG electrodes 806. For example, the detection circuit 810 includes, or otherwise controls, a power source that applies a known voltage (or current) across a pair of the ECG electrodes 806 and detects a resultant current (or voltage) between the pair of the ECG electrodes 806. The impedance is generated based on the applied signal (voltage or current) and the resultant signal (current or voltage). In various cases, the impedance corresponds to respiration of the individual 808, chest compressions performed on the individual 808, and other physiological states of the individual 808. In various examples, the detection circuit 810 includes one or more analog filters configured to filter noise and/or artifact from the resultant signal. The detection circuit 810 generates a digital signal indicative of the impedance using an ADC. This digital signal can be referred to as an "impedance signal" or an "impedance."

The detection circuit 810 provides the ECG signal and/or the impedance signal to one or more processors 812 in the external defibrillator 800. In some implementations, the processor(s) 812 includes a central processing unit (CPU), a graphics processing unit (GPU), both CPU and GPU, or other processing unit or component known in the art.

The processor(s) 812 is operably connected to memory 814. In various implementations, the memory 812 is volatile (such as random access memory (RAM)), non-volatile (such as read only memory (ROM), flash memory, etc.) or some combination of the two. The memory 814 stores instructions that, when executed by the processor(s) 812, causes the processor(s) 812 to perform various operations. In various examples, the memory 814 stores methods, threads, processes, applications, objects, modules, any other sort of executable instruction, or a combination thereof. In some cases, the memory 814 stores files, databases, or a combination thereof. In some examples, the memory 814 includes, but is not limited to, RAM, ROM, electrically erasable programmable read-only memory (EEPROM), flash memory, or any other memory technology. In some examples, the memory 814 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 812 and/or the external defibrillator 800. In some cases, the memory 814 at least temporarily stores the ECG signal and/or the impedance signal.

In various examples, the memory 814 includes a detector 816, which causes the processor(s) 812 to determine, based on the ECG signal and/or the impedance signal, whether the individual 808 is exhibiting a particular heart rhythm. For instance, the processor(s) 812 determines whether the individual 808 is experiencing a shockable rhythm that is treatable by defibrillation. Examples of shockable rhythms include ventricular fibrillation (VF) and ventricular tachycardia (V-Tach). In some examples, the processor(s) 812 determines whether any of a variety of different rhythms (e.g., asystole, sinus rhythm, atrial fibrillation (AF), etc.) are present in the ECG signal.

The processor(s) 812 is operably connected to one or more input devices 818 and one or more output devices 820. Collectively, the input device(s) 818 and the output device(s) 820 function as an interface between a user and the defibrillator 800. The input device(s) 818 is configured to receive an input from a user and includes at least one of a keypad, a cursor control, a touch-sensitive display, a voice input device (e.g., a microphone), a haptic feedback device (e.g., a gyroscope), or any combination thereof. The output device(s) 820 includes at least one of a display, a speaker, a haptic output device, a printer, or any combination thereof. In various examples, the processor(s) 812 causes a display among the input device(s) 818 to visually output a waveform of the ECG signal and/or the impedance signal. In some implementations, the input device(s) 818 includes one or more touch sensors, the output device(s) 820 includes a display screen, and the touch sensor(s) are integrated with the display screen. Thus, in some cases, the external defibrillator 800 includes a touchscreen configured to receive user input signal(s) and visually output physiological parameters, such as the ECG signal and/or the impedance signal.

In some examples, the memory 814 includes an advisor 822, which, when executed by the processor(s) 812, causes the processor(s) 812 to generate advice and/or control the output device(s) 820 to output the advice to a user (e.g., a rescuer). In some examples, the processor(s) 812 provides, or causes the output device(s) 820 to provide, an instruction to perform CPR on the individual 808. In some cases, the processor(s) 812 evaluates, based on the ECG signal, the impedance signal, or other physiological parameters, CPR being performed on the individual 808 and causes the output device(s) 820 to provide feedback about the CPR in the instruction. According to some examples, the processor(s) 812, upon identifying that a shockable rhythm is present in the ECG signal, causes the output device(s) 820 to output an instruction and/or recommendation to administer a defibrillation shock to the individual 808.

The memory 814 also includes an initiator 824 which, when executed by the processor(s) 812, causes the processor(s) 812 to control other elements of the external defibrillator 800 in order to administer a defibrillation shock to the individual 808. The processor(s) 812 can generate the control signal to cause the other elements of the external defibrillator 800 to administer the defibrillation shock. In some examples, the processor(s) 812 executing the initiator 824 selectively causes the administration of the defibrillation shock based on determining that the individual 808 is exhibiting the shockable rhythm and/or based on an input from a user (received, e.g., by the input device(s) 818. In some cases, the processor(s) 812 causes the defibrillation shock to be output at a particular time, which is determined by the processor(s) 812 based on the ECG signal and/or the impedance signal.

The processor(s) 812 is operably connected to a charging circuit 822 and a discharge circuit 824. In various implementations, the charging circuit 822 includes a power source 826, one or more charging switches 828, and one or more capacitors 830. The power source 826 includes, for instance, a battery. The processor(s) 812 initiates a defibrillation shock by causing the power source 826 to charge at least one capacitor among the capacitor(s) 830. For example, the processor(s) 812 activates at least one of the charging switch(es) 828 in the charging circuit 822 to complete a first circuit connecting the power source 826 and the capacitor to be charged. Then, the processor(s) 812 causes the discharge circuit 824 to discharge energy stored in the charged capacitor across a pair of defibrillation electrodes 830, which are in contact with the individual 808. For example, the processor(s) 812 deactivates the charging switch(es) 828 completing the first circuit between the capacitor(s) 830 and the power source 826, and activates one or more discharge switches 832 completing a second circuit connecting the charged capacitor 830 and at least a portion of the individual 808 disposed between defibrillation electrodes 834. For example, the processor(s) 812 generates the control signal to deactivates the charging switch(es) 828 and activate the one or more discharge switches 832.

The energy is discharged from the defibrillation electrodes 834 in the form of a defibrillation shock. For example, the defibrillation electrodes 834 are connected to the skin of the individual 808 and located at positions on different sides of the heart of the individual 808, such that the defibrillation shock is applied across the heart of the individual 808. The defibrillation shock, in various examples, depolarizes a significant number of heart cells in a short amount of time. The defibrillation shock, for example, interrupts the propagation of the shockable rhythm (e.g., VF or V-Tach) through the heart. In some examples, the defibrillation shock is 200 J or greater with a duration of about 0.015 seconds. In some cases, the defibrillation shock has a multiphasic (e.g., biphasic) waveform. The discharge switch(es) 832 are controlled by the processor(s) 812, for example. In various implementations, the defibrillation electrodes 834 are connected to defibrillation leads 836. The defibrillation leads 836 are connected to a defibrillation port 838, in implementations. According to various examples, the defibrillation leads 836 are removable from the defibrillation port 838. For example, the defibrillation leads 836 are plugged into the defibrillation port 838.

In various implementations, the processor(s) 812 is operably connected to one or more transceivers 840 that transmit and/or receive data over one or more communication networks 842. For example, the transceiver(s) 840 includes a network interface card (NIC), a network adapter, a local area network (LAN) adapter, or a physical, virtual, or logical address to connect to the various external devices and/or systems. In various examples, the transceiver(s) 840 includes any sort of wireless transceivers capable of engaging in wireless communication (e.g., radio frequency (RF) communication). For example, the communication network(s) 842 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 840 includes other wireless modems, such as a modem for engaging in WI-FI^{®}, WIGlG^{®}, WIMAX^{®}, BLUETOOTH^{®}, NFC, radio frequency identification (RFID), or infrared communication over the communication network(s) 842.

The defibrillator 800 is configured to transmit and/or receive data (e.g., ECG data, impedance data, data indicative of one or more detected heart rhythms of the individual 808, data indicative of one or more defibrillation shocks administered to the individual 808, etc.) with one or more external devices 844 via the communication network(s) 842. The external devices 844 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 842. In some examples, the external device(s) 844 is located remotely from the defibrillator 800, such as at a remote clinical environment (e.g., a hospital). According to various implementations, the processor(s) 812 causes the transceiver(s) 840 to transmit data to the external device(s) 844. In some cases, the transceiver(s) 840 receives data from the external device(s) 844 and the transceiver(s) 840 provide the received data to the processor(s) 812 for further analysis.

In some cases, the external device(s) 844 include one or more medical devices. According to various implementations, the memory 814 further includes a coordinator 846 which, when executed by the processor(s) 812, causes the processor(s) 812 to coordinate with the external device(s) 844, such as by generating a control signal configured to cause administering therapy (e.g., defibrillation, pacing, etc.) to a subject based on communication between the defibrillator 800 and the external device(s) 844, as described herein. In some implementations, the processor(s) 812, when executing the coordinator 846, receives data 114 from the external device(s) 844, analyzes the data 114 to determine a control parameter(s), and generates a control signal configured to cause administering therapy (e.g., defibrillation, pacing, etc.) in accordance with the control parameter(s), as described herein. In some implementations, the processor(s) 812, when executing the coordinator 846, determines a parameter associated with the therapy being administered by the defibrillator 800, such as a physiological parameter of the individual 806, determines, by analyzing the parameter, a control parameter for controlling administration of therapy to the individual 806 by the external device(s) 844, and sends data (e.g., via the transceiver(s) 840) to the external device(s) 844, the data representing the control parameter. In general, the coordinator 846, when executed by the processor(s) 812, may cause the processor(s) 812 to perform any of the processes 400, 500, 600, and/or 700 described herein.

In various implementations, the external defibrillator 800 also includes a housing 846 that at least partially encloses other elements of the external defibrillator 800. For example, the housing 846 encloses the detection circuit 810, the processor(s) 812, the memory 814, the charging circuit 822, the transceiver(s) 840, or any combination thereof. In some cases, the input device(s) 818 and output device(s) 820 extend from an interior space at least partially surrounded by the housing 846 through a wall of the housing 846. In various examples, the housing 846 acts as a barrier to moisture, electrical interference, and/or dust, thereby protecting various components in the external defibrillator 800 from damage.

In some implementations, the external defibrillator 800 is an automated external defibrillator (AED) operated by an untrained user (e.g., a bystander, layperson, etc.) and can be operated in an automatic mode. In automatic mode, the processor(s) 812 automatically identifies a rhythm in the ECG signal, makes a decision whether to administer a defibrillation shock, charges the capacitor(s) 830, generates a control signal to discharge the capacitor(s) 830, or any combination thereof. In some cases, the processor(s) 812 controls the output device(s) 820 to output (e.g., display) a simplified user interface to the untrained user. For example, the processor(s) 812 refrains from causing the output device(s) 820 to display a waveform of the ECG signal and/or the impedance signal to the untrained user, in order to simplify operation of the external defibrillator 800.

In some examples, the external defibrillator 800 is a monitor-defibrillator utilized by a trained user (e.g., a clinician, an emergency responder, etc.) and can be operated in a manual mode or the automatic mode. When the external defibrillator 800 operates in manual mode, the processor(s) 812 cause the output device(s) 820 to display a variety of information that may be relevant to the trained user, such as waveforms indicating the ECG data and/or impedance data, notifications about detected heart rhythms, and the like.

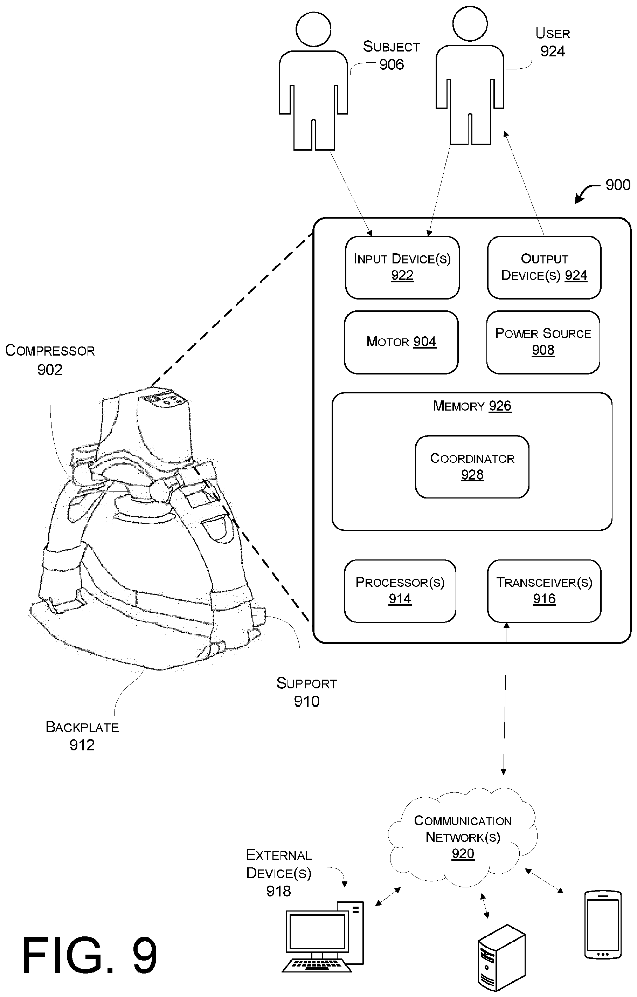

FIG. 9 illustrates a chest compression device 900 configured to perform various functions described herein. For example, the chest compression device 900 is the monitoring device 106, the treating device 110, the monitoring device 202, the treating device 204, the first medical device 302, the second medical device 304, the intermediary device 210, or the intermediary device 306 described above with reference to FIGS. 1A to 3.

In various implementations, the chest compression device 900 includes a compressor 902 that is operatively coupled to a motor 904. The compressor 902 is configured to physically administer a force to the chest of a subject 906 that compresses the chest of the subject 906. In some examples, the compressor 902 includes at least one piston that periodically moves between two positions (e.g., a compressed position and a release position) at a compression frequency. For example, when the piston is positioned on the chest of the subject 906, the piston compresses the chest when the piston is moved into the compressed position. A suction cup may be positioned on a tip of the piston, such that the suction cup contacts the chest of the subject 906 during operation. In various cases, the compressor 902 includes a band that periodically tightens to a first tension and loosens to a second tension at a compression frequency. For instance, when the band is disposed around the chest of the subject 906, the band compresses the chest when the band tightens.

The motor 904 is configured to convert electrical energy stored in a power source 908 into mechanical energy that moves and/or tightens the compressor 902, thereby causing the compressor 902 to administer the force to the chest of the subject 906. In various implementations, the power source 908 is portable. For instance, the power source 908 includes at least one rechargeable (e.g., lithium-ion) battery. In some cases, the power source 908 supplies electrical energy to one or more elements of the chest compression device 900 described herein.

In various cases, the chest compression device 900 includes a support 910 that is physically coupled to the compressor 902, such that the compressor 902 maintains a position relative to the subject 906 during operation. In some implementations, the support 910 is physically coupled to a backplate 912, cot, or other external structure with a fixed position relative to the subject 906. According to some cases, the support 910 is physically coupled to a portion of the subject 906, such as wrists of the subject 906.

The operation of the chest compression device 900 may be controlled by at least one processor 914. In various implementations, the motor 906 is communicatively coupled to the processor(s) 914. Specifically, the processor(s) 914 is configured to output a control signal to the motor 906 that causes the motor 906 to actuate the compressor 902. For instance, the motor 906 causes the compressor 902 to administer the compressions to the subject 906 based on the control signal. In some cases, the control signal indicates one or more treatment parameters of the compressions. Examples of treatment parameters include a frequency, timing, duty cycle, depth, force, position, velocity, height, and acceleration of the compressor 902 administering the compressions. According to various cases, the control signal causes the motor 906 to cease compressions.

In various implementations, the chest compression device 900 includes at least one transceiver 916 configured to communicate with at least one external device 918 over one or more communication networks 920. Any communication network described herein can be included in the communication network(s) 920 illustrated in FIG. 9. The external device(s) 918, for example, includes at least one of a monitor-defibrillator, an AED, an ECMO device, a ventilation device, a patient monitor, a mobile phone, a server, or a computing device. In some implementations, the transceiver(s) 916 is configured to communicate with the external device(s) 918 by transmitting and/or receiving signals wirelessly. For example, the transceiver(s) 916 includes a NIC, a network adapter, a LAN adapter, or a physical, virtual, or logical address to connect to the various external devices and/or systems. In various examples, the transceiver(s) 916 includes any sort of wireless transceivers capable of engaging in wireless communication (e.g., RF communication). For example, the communication network(s) 920 includes one or more wireless networks that include a 3GPP network, such as an LTE RAN (e.g., over one or more LTE bands), an NR RAN (e.g., over one or more NR bands), or a combination thereof. In some cases, the transceiver(s) 916 includes other wireless modems, such as a modem for engaging in WI-FI^{®}, WIGIG^{®}, WIMAX^{®}, BLUETOOTH^{®}, NFC, RFID, or infrared communication over the communication network(s) 920. The signals, in various cases, encode data in the form of data packets, datagrams, or the like. In some cases, the signals are transmitted as compressions are being administered by the chest compression device 900 (e.g., for real-time feedback by the external device(s) 918), after compressions are administered by the chest compression device 900 (e.g., for post-event review at the external device 918), or a combination thereof.

In various cases, the processor(s) 914 generates the control signal based on data encoded in the signals received from the external device(s) 918. For instance, the signals include an instruction to initiate the compressions, and the processor(s) 914 instructs the motor 906 to begin actuating the compressor 902 in accordance with the signals.

In some cases, the chest compression device 900 includes at least one input device 922. In various examples, the input device(s) 922 is configured to receive an input signal from a user 924, who may be a rescuer treating the subject 906. Examples of the input device(s) 922 include, for instance, at a keypad, a cursor control, a touch-sensitive display, a voice input device (e.g., a microphone), a haptic feedback device (e.g., a gyroscope), or any combination thereof. In various implementations, the processor(s) 914 generate the control signal based on the input signal. For instance, the processor(s) 914 generate the control signal to adjust a frequency of the compressions based on the chest compression device 900 detecting a selection by the user 924 of a user interface element displayed on a touchscreen or detecting the user 924 pressing a button integrated with an external housing of the chest compression device 900.

According to some examples, the input device(s) 922 include one or more sensors. The sensor(s), for example, is configured to detect a physiological parameter of the subject 906. In some implementations, the sensor(s) is configured to detect a state parameter of the chest compression device 900, such as a position of the compressor 902 with respect to the subject 906 or the backplate 912, a force administered by the compressor 902 on the subject 906, a force administered onto the backplate 922 by the body of the subject 906 during a compression, or the like. According to some implementations, the signals transmitted by the transceiver(s) 916 indicate the physiological parameter(s) and/or the state parameter(s).

The chest compression device 900 further includes at least one output device 924, in various implementations. Examples of the output device(s) 924 include, for instance, least one of a display (e.g., a projector, an LED screen, etc.), a speaker, a haptic output device, a printer, or any combination thereof. In some implementations, the output device(s) 924 include a screen configured to display various parameters detected by and/or reported to the chest compression device 900, a charge level of the power source 908, a timer indicating a time since compressions were initiated or paused, and other relevant information.

The chest compression device 900 further includes memory 926. In various implementations, the memory 926 is volatile (such as random access memory (RAM)), non-volatile (such as read only memory (ROM), flash memory, etc.) or some combination of the two. The memory 926 stores instructions that, when executed by the processor(s) 914, causes the processor(s) 914 to perform various operations. In various examples, the memory 926 stores methods, threads, processes, applications, objects, modules, any other sort of executable instruction, or a combination thereof. In some cases, the memory 926 stores files, databases, or a combination thereof. In some examples, the memory 926 includes, but is not limited to, RAM, ROM, EEPROM, flash memory, or any other memory technology. In some examples, the memory 926 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 926 stores instructions, programs, threads, objects, data, or any combination thereof, that cause the processor(s) 914 to perform various functions. In various cases, the memory 926 stores one or more parameters that are detected by the chest compression device 900 and/or reported to the chest compression device 900.

In some cases, the external device(s) 918 include one or more medical devices. According to various implementations, the memory 926 also stores instructions for executing a coordinator 928,which, when executed by the processor(s) 914, causes the processor(s) 914 to coordinate with the external device(s) 918. The coordination with the external device(s) 918 may include generating a control signal to cause administering therapy (e.g., chest compressions) to a subject 906 based on communication between the chest compression device 900 and the external device(s) 918, as described herein. In some implementations, the processor(s) 914, when executing the coordinator 928, receives data 114 from the external device(s) 918, analyzes the data 114 to determine a control parameter(s), and generates a control signal configured to cause administering therapy (e.g., chest compressions) in accordance with the control parameter(s), as described herein. In some implementations, the processor(s) 914, when executing the coordinator 928, determines a parameter associated with the therapy being administered by the chest compression device 900, such as a physiological parameter of the subject 906, determines, by analyzing the parameter, a control parameter for controlling administration of therapy to the subject 906 by the external device(s) 918, and sends data (e.g., via the transceiver(s) 916) to the external device(s) 918, the data representing the control parameter. In general, the coordinator 928, when executed by the processor(s) 914, may cause the processor(s) 914 to perform any of the processes 400, 500, 600, and/or 700 described herein.

### CONCLUSION

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; 16% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by as long as they fall within the scope of the claims.

## Claims

1. An external defibrillator (800) comprising:
a transceiver (840);
a discharge circuit (824) configured to output an electrical shock to a subject; and
a processor (812) configured to:
receive, via the transceiver (840), data from a mechanical chest compression device (900);
determine, by analyzing the data, a timing for outputting an electrical shock to the subject in coordination with chest compressions that are being administered to the subject by the mechanical chest compression device (900); and
cause the discharge circuit (824) to output the electrical shock to the subject in accordance with the timing;
further comprising an output device (820) configured to output information, **characterized in that** the processor (812) is further configured to:
determine, by analyzing the data, a time of an upcoming pause that will follow a chest compression administered to the subject by the mechanical chest compression device (900);
detect a physiological parameter of the subject only during the time; and
cause the output device (820) to output the physiological parameter.

2. The external defibrillator of claim 1, wherein the electrical shock comprises a defibrillation shock.

3. The external defibrillator of claim 1 or 2, wherein the processor (812) is further configured to:
determine, by analyzing the data, a medication for the subject; and
cause the output device (820) to output an indication of the medication.

4. The external defibrillator of any of claims 1-3, wherein the processor (812) is further configured to:
determine, by analyzing the data, a depth to which the chest compressions are being administered; and
cause the output device (820) to output an indication of the depth.

5. The external defibrillator of any of claims 1-4, wherein the processor (812) is further configured to:
determine, by analyzing the data, a force with which the chest compressions are being administered; and
cause the output device (820) to output an indication of the force.

## Patentansprüche

1. Externer Defibrillator (800), umfassend:
einen Transceiver (840);
eine Entladeschaltung (824), die dazu konfiguriert ist; einen elektrischen Schock an einen Probanden auszugeben; und einen Prozessor (812), der konfiguriert ist zum:
Empfangen von Daten von einer mechanischen Thoraxkompressionsvorrichtung (900) mittels des Transceivers (840) zu empfangen;
Bestimmen einer Zeitvorgabe für das Ausgeben eines elektrischen Schocks an den Probanden in Koordination mit Thoraxkompressionen, die dem Probanden durch die mechanische Thoraxkompressionsvorrichtung (900) verabreicht werden, durch Analysieren der Daten; und
Bewirken, dass die Entladeschaltung (824) den elektrischen Schock an den Probanden gemäß der Zeitvorgabe ausgibt;
ferner umfassend eine Ausgabevorrichtung (820), die dazu konfiguriert ist, Informationen auszugeben, **dadurch gekennzeichnet, dass** der Prozessor (812) ferner konfiguriert ist zum:
Bestimmen einer Zeit einer bevorstehenden Pause, die auf eine Thoraxkompression folgen wird, die dem Probanden durch die mechanische Thoraxkompressionsvorrichtung (900) verabreicht wird, durch Analysieren der Daten;
Erfassen eines physiologischen Parameters des Probanden nur während der Zeit; und
Bewirken, dass die Ausgabevorrichtung (820) den physiologischen Parameter ausgibt.

2. Externer Defibrillator nach Anspruch 1, wobei der elektrische Schock einen Defibrillationsschock umfasst.

3. Externer Defibrillator nach Anspruch 1 oder 2, wobei der Prozessor (812) ferner konfiguriert ist zum:
Bestimmen einer Medikation für den Probanden durch Analysieren der Daten; und
Bewirken, dass die Ausgabevorrichtung (820) eine Angabe der Medikation ausgibt.

4. Externer Defibrillator nach einem der Ansprüche 1 bis 3, wobei der Prozessor (812) ferner konfiguriert ist zum:
Bestimmen einer Tiefe, zu der die Thoraxkompressionen verabreicht werden, durch Analysieren der Daten; und
Bewirken, dass die Ausgabevorrichtung (820) eine Angabe der Tiefe ausgibt.

5. Externer Defibrillator nach einem der Ansprüche 1 bis 4, wobei der Prozessor (812) ferner konfiguriert ist zum:
Bestimmen einer Kraft, mit der die Thoraxkompressionen verabreicht werden, durch Analysieren der Daten; und
Bewirken, dass die Ausgabevorrichtung (820) eine Angabe der Kraft ausgibt.

## Revendications

1. Défibrillateur externe (800) comprenant:
un émetteur-récepteur (840);
un circuit de décharge (824) configuré pour délivrer un choc électrique à un sujet; et
un processeur (812) configuré pour:
recevoir, par l'intermédiaire de l'émetteur-récepteur (840), des données provenant d'un dispositif de compression thoracique mécanique (900);
déterminer, par l'analyse des données, un moment pour délivrer un choc électrique au sujet en coordination avec les compressions thoraciques qui sont administrées au sujet par le dispositif de compression thoracique mécanique (900); et
amener le circuit de décharge (824) à délivrer le choc électrique au sujet conformément au moment;
comprenant en outre un dispositif de délivrance (820) configuré pour délivrer des informations, **caractérisé en ce que** le processeur (812) est configuré en outre pour:
déterminer, par l'analyse des données, un moment d'une pause à venir qui suivra une compression thoracique administrée au sujet par le dispositif de compression thoracique mécanique (900);
détecter un paramètre physiologique du sujet uniquement pendant ce moment; et
amener le dispositif de délivrance (820) à délivrer le paramètre physiologique.

2. Défibrillateur externe selon la revendication 1, dans lequel le choc électrique comprend un choc de défibrillation.

3. Défibrillateur externe selon la revendication 1 ou 2, dans lequel le processeur (812) est configuré en outre pour:
déterminer, par l'analyse des données, un médicament pour le sujet; et
amener le dispositif de délivrance (820) à délivrer une indication du médicament.

4. Défibrillateur externe selon l'une quelconque des revendications 1 à 3, dans lequel le processeur (812) est configuré en outre pour:
déterminer, par l'analyse des données, une profondeur à laquelle les compressions thoraciques sont administrées; et
amener le dispositif de délivrance (820) à délivrer une indication de la profondeur.

5. Défibrillateur externe selon l'une quelconque des revendications 1 à 4, dans lequel le processeur (812) est configuré en outre pour:
déterminer, par l'analyse des données, une force avec laquelle les compressions thoraciques sont administrées; et
amener le dispositif de délivrance (820) à délivrer une indication de la force.
